# EUROPEAN PATENT SPECIFICATION

(11) **EP 0 244 803 B1**
(45) Date of publication and mention of the grant of the patent: **26.10.1994**
(21) Application number: 87106440.8
(22) Date of filing: 04.05.1987
(51) Int. Cl.: C07D 409/04, C07D 405/04, C07D 401/04, C07D 403/04, C07D 417/04, C07D 409/06, C07D 405/06, C07D 401/06, C07D 403/06, C07D 417/06, C07D 233/84

(54) **Novel dopamine beta hydroxylase inhibitors**
Dopamin-beta-hydroxylasehemmer
Inhibiteurs de dopamine-bêta-hydroxylase

(30) Priority: 06.05.1986 US 860263
(43) Date of publication of application: 11.11.1987
(73) Proprietor: MERRELL DOW PHARMACEUTICALS INC., Cincinnati Ohio 45215-6300 (US)
(72) Inventor: Matthews, Donald P., Indianapolis Indiana 46240 (US); McCarthy, James R., Zionsville Indiana 46077 (US); Whitten, Jeffrey P., Zionsville Indiana 46077 (US); Broersma, Robert J., Jr., Noblesville Indiana 46060 (US)
(74) Representative: Sgarbi, Renato

(56) References cited:
- EP-A- 0 015 155
- EP-A- 0 115 640
- EP-A- 0 125 033
- EP-A- 0 125 783
- EP-A- 0 214 740
- DE-A- 312 877
- DE-A- 2 903 653
- DE-A- 3 228 266
- FR-A- 2 073 427
- GB-A- 2 028 317
- US-A- 3 505 350
- Fuller, R.W. et al.: AVD. ENZYME REGUL., vol. 15, 1976, pages 267-281.
- Chemical Abstracts, vol. 88, no. 17, 24 April 1978, page 535, abstract no. 121049y, Columbus, Ohio, US; R.J. Sundberg et Al.
- Chemical Abstracts, vol. 107, no. 18, 2 November 1987, page 757, abstract no. 165513k, Columbus Ohio, US.
- Chemical Abstracts, vol. 58, no. 8, 15 April 1963, page 7922, Columbus , Ohio, US; I.I. KOVTUNOVSKAJA-LEVSHINA
- Chemical Abstracts, vol. 100, no.11, 12 March 1984, page 540, abstract no. 85629t, Columbus, Ohio, US; E. BELGODERE et al.:
- Chemical Abstracts, vol. 86, no. 19, 9 May 1977, page 555, abstract no. 139941b, Columbus, Ohio, US; E. REGEL et al.

## Description

This invention relates to novel derivatives of 1-substituted imidazoles, to the processes and intermediates useful for their preparation, to the pharmaceutical compositions containing said imidazoles, to their dopamine beta-hydroxylase inhibiting pharmacological activity and to their applied use in the treatment of hypertension.

More specifically, this invention relates to novel 1-imidazole derivatives of the formula 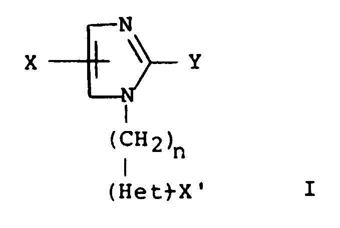
including the 2-thione tautomers thereof, and the non-toxic pharmaceutically acceptable salts thereof, wherein n is zero or the integer 1, 2, 3 or 4, X is hydrogen, cyano, C₁₋₆ lower alkyl, chloro, bromo, phenyl, or benzyl, Z-substituted phenyl or benzyl with Z being C₁₋₆ lower alkyl or halogeno; Y is aminomethyl, amido, alpha-keto acid and their C₁₋₆ esters, thioamido, amidino, aminoethylthio, or sulfhydryl, with the proviso that when Y is sulfhydryl or aminoethylthio, then X is hydrogen; (Het)̵X' is a heterocyclic moiety of the group thienyl, furyl, pyrazolyl, pyrimidinyl, pyrrolyl, and imidazol-2-yl, optionally substituted with either a halogen or a C₁₋₆ lower alkyl, for use as a medicine. Said compounds possess dopamine betahydrolase (DBH) inhibiting properties and are useful in the treatment of hypertension. Another object of the present invention is represented by 1-imidazole derivatives of the above formula I, wherein X, Y, (Het)X' and n have the meanings reported above, with the proviso that when Y is SH and Het is 2-thienyl, then n is other than one.

The compound, of the above formula I wherein n is one, X and X' represents hydrogen, Y represents sulfhydryl, and Het represents 2-thienyl, i.e. 1,3-dihydro-1-(2-thienylmethyl)-2H-imidazole-2-thione, was disclosed by E. Belgodere et al. in Heterocycles, 20, 2019-2023 (1983).

R.W.FULLER et al. (ADV. ENZYME REGUL. Vol. 15, 1976, 267-281) discloses 2-mercapto-imidazole derivatives bearing a (C₁-C₈)alkyl or a cyclohexyl group at position 1; EP-A-0125033 discloses 2-mercapto-imidazole derivatives bearing hydrogen or (C₁-C₄)alkyl at position 2 and a substituted phenyl or substituted phenyl (C₁-C₄)alkyl group at position 1; and EP-A-0125783 discloses imidazole derivatives bearing a carboxy group or a -CH₂NHR' group, wherein R' is hydrogen, phenyl or benzyl, at position 2, and a substituted phenyl or substituted phenyl (C₁-C₄)alkyl, at position 1. These classes of imidazole derivatives are reported to possess dopamine betahydroxylase inhibiting properties.

As used herein the term "lower alkyl" includes straight, branched-chain or cyclized hydrocarbyl radicals having up to six carbon atoms, preferably methyl, ethyl and propyl; halogeno preferably includes chloro, fluoro or bromo; Z substituted phenyl or benzyl includes those substituents at the ortho or meta positions, but preferably are located at the para- position. "Aminomethyl" includes those moieties of the formula -CH₂NR₁R₂, "amido" includes those moieties of the formula -CONHR₁; "thioamido" includes those moieties having the structure -CSNHR₁; "amidino" includes those moieties of the formula R₃N=C-NR₁R₂ with R₃ being H or OH; "sulfhydryl" includes -SR₁; alpha-keto acid and esters include -COCOOR₁, "aminoethylthio" includes those moieties of the formula -SCH₂CH₂NR₁R₂, wherein, in each of the foregoing instances, R₁ and R₂ independently represent hydrogen or C₁₋₆ lower alkyl. The terms represented by "Het" of formula I embraces those named heterocyclics wherein "thienyl" includes its 2- and 3-position isomers, "furyl" includes its 2- and 3- position isomers, pyrimidinyl includes its 2-, 4- and 5- position isomers, "pyrrolyl" includes its 2- and 3- position isomers and their 2,5-dihydro 1H-pyrrolyl analogs, "pyrazolyl" includes its 3-, 4- and 5- isomers and its 4,5-dihydro analogs. The heterocyclic moieties may also contain halogeno or lower alkyl substituents at any of their open alkyl substituents at any of their open positions, i.e., X' is hydrogen, halogeno or C₁₋₆ lower alkyl. In those instances wherein n is zero, the heterocyclic moiety is attached directly to the nitrogen atom of the imidazole moiety, otherwise, it is separated by an alkylene bridging moiety having up to four carbon atoms, preferably one or three, i.e., methylene or propylene.

The compounds of formula I are useful in the free base form and, except in those instances wherein Y is SH, in the form of their acid addition salts; both forms being within the purview of this invention. The acid addition salts are simply a more convenient form for use and, in practice, use of the salt amounts to use of the free base. The acids which can be used include those which produce, when combined with the free base, pharmaceutically acceptable salts, that is, salts whose anions are relatively innocuous to the animal organism in pharmaceutical doses of the salts. In practice, it is convenient to form sulfate, phosphate, methansulfonate or lactate salts. Others are those derived from mineral acids (e.g., hydrochloric), and organic acids such as acetic acid, citric acid, tartaric acid, ethanesulfonic acid, benzenesulfonic acid, p-toluenesulfonic acid and the like. The acid salts are prepared by standard techniques such as by dissolving the free base in aqueous or aqueous-alcohol solution or other suitable solvents containing the appropriate acid and isolating by evaporating the solution, or by reacting the free base and in an organic solvent in which case the salt separates directly or can be obtained by concentration of the solution.

In the preparation of the compounds of this invention it is quite obvious that the specific compound sought to be prepared will have a bearing of the particular process path to be utilized. Such factors as the specific X, X', Z and/or Y substituents, the presence or absence of an alkylene bridge between the imidazolyl moiety and its attached heterocycle, and ready availability of the starting materials all play a role in choosing the specific path to be followed in the preparation of the compounds of this invention. Those factors are readily appreciated by one of ordinary skill in the art. However, in general, the compounds of this invention may be prepared by standard techniques and processes analogously known in the art.

In those instances wherein the compounds sought to be prepared contain a sulfhydryl substituent on the imidazole ring moiety, and n is either zero or an alkylene bridge, it is convenient to react an isothiocyanate derivative (II) with an appropriate acetal (III) to form a reaction product (IV), which is subjected to a cyclization reaction to form the imidazole ring bearing the sulfhydryl substituent (Ia). The sulfhydryl substituent may readily be converted to its aminoethylthio derivative (Ib) by reaction with an aminoethylchloride. These reactions are depicted in Reaction Scheme A. 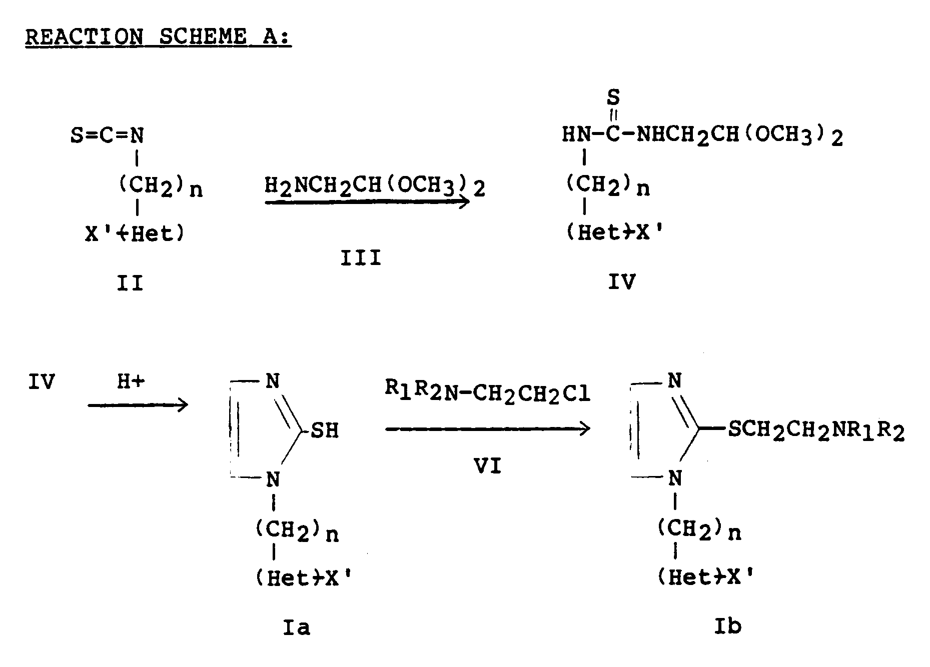
wherein Het, n, X, X', R₁ and R₂ are as defined in formula I.

Of course, in those instances wherein the desired product contains an X substituent other than halogen, the acetal (III) bears the appropriate substituent. In those instances wherein it is desired to prepare a halogenated derivative, i.e., X is halogeno, then the thione (Ia) is appropriately protected and halogenated according to procedures well known in the art.

The reaction of the isothiocyanate derivatives (II) with the acetal (III) is a simple condensation reaction, preferably effected by heating the reactants under reflux conditions using inert solvents, e.g., toluene or DMF at 80°C, to form the thiourea (IV) intermediates. These intermediates are subjected to cyclization by treatment with acid, preferably by refluxing the intermediates with aqueous hydrochloric acid in ethanol to produce the desired 1-substituted-2-imidazole bearing a sulfhydryl substituent (Ia). The sulfhydryl moiety of the compounds may be converted to its aminoethylthio analog (Ib) by treatment with the appropriate aminoethyl halide, as its hydrohalic salts, preferably by admixture at room temperature in the presence of a solvent. Alternatively, the sulfhydryl moiety may be chemically removed by catalytic reduction, preferably utilizing Raney nickel or dilute nitric acid at 80°C to 90°C or other equivalently functional systems. In this instance, the chemically reduced product may be converted to its 2-cyanoimidazole derivative by sequential treatment with cyanogen chloride and a base according to standard procedures well known in the art.

Alternatively, in those instances wherein n is other than zero, the 1-hetero-2-imidazoles (Ic) may be prepared by treating a heteroaldehyde derivative (V) with the aforementioned acetals (III) to form a Schiff base which is reduced to form an intermediate (VI) which is subjected to a cyclization reaction by treatment with aqueous HCl in ethanol in the presence of an alkali metal isothiocyanate preferably KSCN. These reactions may be depicted by the following reaction scheme. 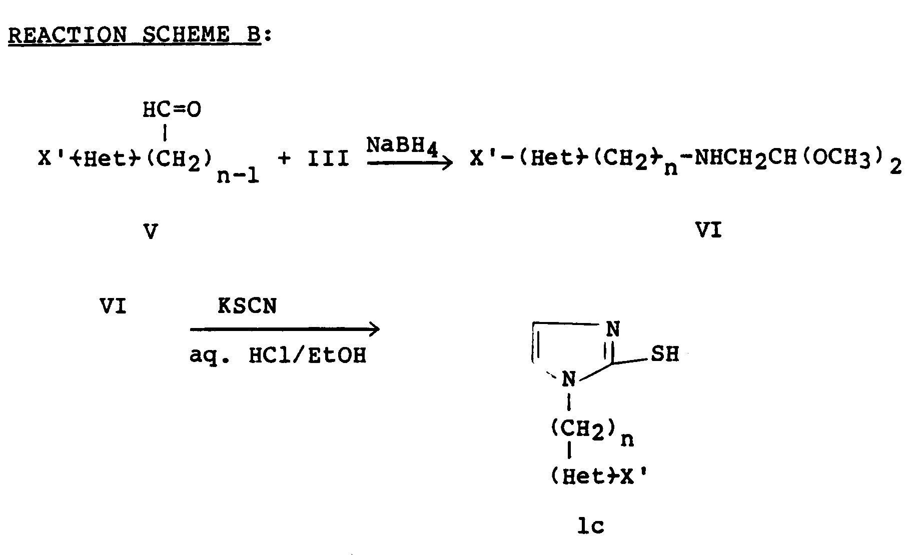
wherein (Het)̵X', is as previously defined and n is other than zero.

Still another alternate method for preparing compounds of Formula I is by reacting a halo derivative of an appropriate heterocycle with an X-substituted imidazole to form a X'(̵het)̵(CH₂)ₙ substituted-X-substituted-imidazole. In one illustration of this type reaction the imidazole is first acylated and then the N-acylated imidazole is treated with a halo derivative of an appropriate heterocyclic to form an intermediate N'-acylated-N³-imidazolium cation which is hydrolized to form the appropriately X-substituted analogs of Id. In another illustration the alkali metal derivatives of an X-substituted imidazole may be reacted with a halo derivative of an appropriate heterocycle to produce the desired 1-(2(CH₂)ₙ-heterocycle)-X-substituted imidazole; the reaction being effected according to standard and well-known conditions. These reactions are depicted by the following reaction scheme. 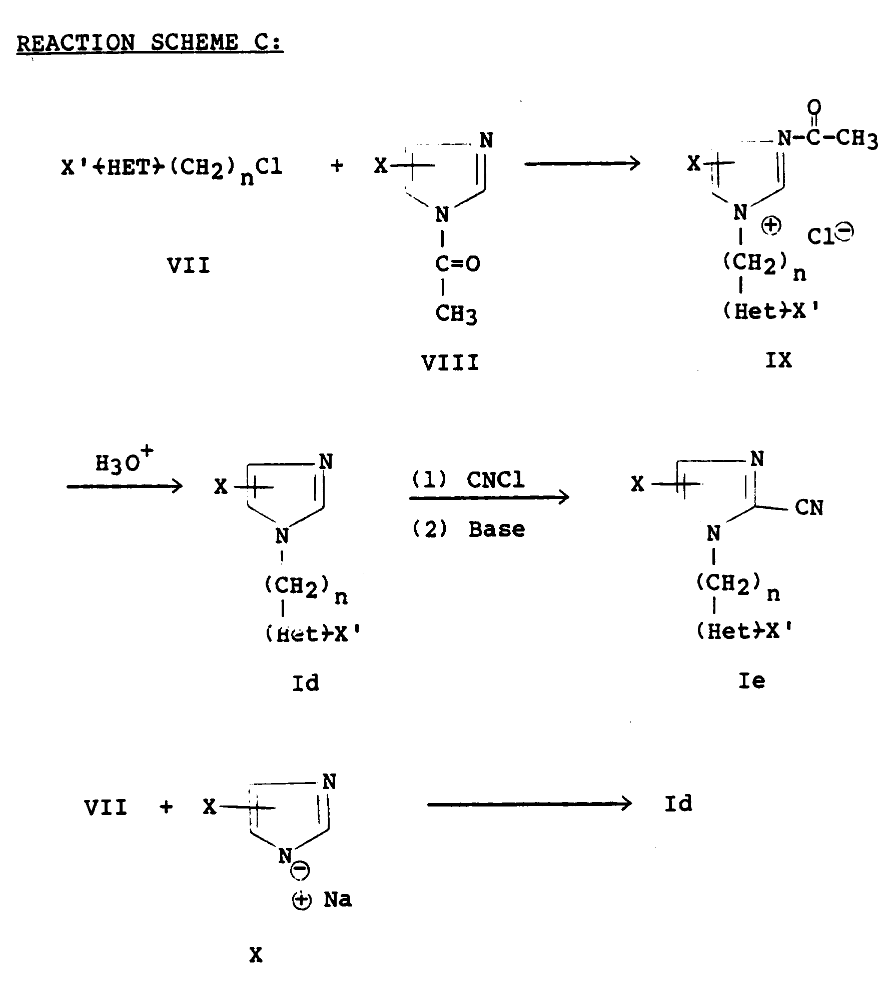
wherein the X', X and n moieties are as previously defined.

Useful references for some of the foregoing reactions are J. Med. Chem. 28, 1405-1413 (1985); J. Med. Chem. 10, 1409 (1985); J. Med. Chem. 18, 833 (1975) and Chem. Rev. 390 (1944).

The compounds prepared by the foregoing reaction schemes may readily be converted to the desired 2-Y'-X-substituted-2H-imidazole (Y' being as defined for Y except that the -SR₁ and -SCH₂CH₂NR₁R₂ moieties are excluded), by standard procedures well known in the art. In effecting these transformations it is convenient to utilize the 2-cyano derivative of the imidazoles of formula Id as starting materials for the initiation of these conversions.

The cyano derivatives may readily be prepared by reacting the imidazole (Id) with cyanogen chloride, in a nitrogen atmosphere, in an solvent, preferably acetonitrile or toluene, at room temperature and then treating the reaction product with a base, preferably triethylamine at below 0° C temperatures. These procedures are standard and well known in the art.

The cyano moiety may readily be converted to its aminomethyl derivatives by standard reduction procedures (i.e., LiAlH₄, or H₂/PtO₂, or H₂/Pd/C/HCl) well known reducing reagents for this conversion. Alkylation of the aminomethyl moiety (-CH₂NH₂) may be effected by use of the Borsch reduction in the presence of the appropriate aldehyde (i.e., reacting RCHO/NaCNBH₃ in ethanol at room temperature at pH 4.0-5.0). The imidate ester (Y is 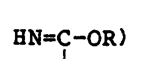
is prepared by reacting the appropriate alcohol with the cyano derivative. The imidate ester 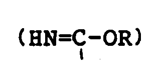
is readily converted to its amidino derivative (Y is 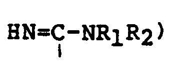
by reaction with the appropriate amine in acid, and to its hydroxy amidine (Y is 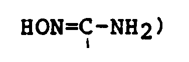
by reaction with hydroxylamine in the presence of 1 equivalent of acid in an alcoholic solvent. This derivative can be converted to its alkylated derivative (i.e., Y is 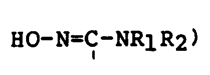
by treatment with an amine in the presence of 1 equivalent of acid. Treatment of the amidine (Y is 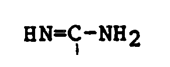
or 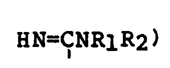
with H₂S in pyridine (with warming) yields the corresponding thioamides (Y is 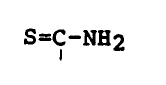
or 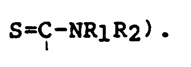
Alternatively the cyano moiety may be treated with H₂S in pyridine to yield the desired thioamide (Y is 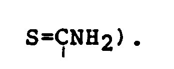
Acid catalysis of the nitrile yields the amide (Y is -CONH₂).

The following examples merely illustrate the various techniques and procedures utilized for the preparation of the compounds of this invention; it being understood that they are not meant to limit the scope of the compounds defined by this invention.

### EXAMPLE 1

### 1,3-Dihydro-1-(2-thienylmethyl)-2H-imidazole-2-thione

A mixture of 33.6 g (0.3 mol) thiophene-2-carboxaldehyde, 39.9 g (0.3 mol) aminoacetaldehyde diethyl acetal, 0.3 g TsOH and 200 ml ethanol is placed in a 500 ml flask and heated to reflux. After 2 h, the reaction is concentrated and the residue dissolved in 250 ml ethanol. Solid NaBH₄ (12.5 g, 0.33 mol) is added in small portions. The reaction is refluxed for 1-1/2 h, cooled to room temperature and poured into cold water. The product is extracted into CH₂Cl₂ (2 X 250 ml). After drying (Na₂SO₄) and concentration 66.7 g crude product is obtained as a pale yellow oil. 22.9 g (0.1 mol) of the crude amine is placed in a 500 ml flask along with 11.7 g (0.12 mol) KSCN, 150 ml ethanol, 40 ml water and 15 ml concentrated hydrochloric acid. After refluxing for 5 hrs., the reaction is poured onto 1 l ice water. The white crystals are collected and dried to give 12.0 g (61%) product, mp 128-130°C (EtOH).

### EXAMPLE 2

### 1,3-Dihydro-1-(1-methylpyrrol-2-ylmethyl)-2H-imidazole-2-thione

Procedure: Reflux a mixture of 10.9 g (0.1 mol) 1-methyl-2-pyrrolecarboxaldehyde, 13.1 g (0.1 mol) aminoacetaldehyde diethylacetal 0.1 g TsOH·H₂O and 200 ml ethanol for 2 h. Cool and concentrate the resulting mixture to obtain a tan oil. Dissolve the residue in 200 ml ethanol and slowly add 4.2 g (0.11 mol) solid NaBH₄. After addition is completed, reflux the reaction mixture for 2 h, pour the cooled reaction mixture into 1 l water. Extract the product into CH₂Cl₂ (2X 200 ml), dry (Na₂SO₄) and concentrate to obtain 24.2 g of crude acetal derivative. Mix the acetal derivative (22.6 g) (0.01 mol) with 11.6 g (0.12 mol) KSCN, 150 ml ethanol, 40 ml H₂O and 15 ml concentrated hydrochloric acid, reflux the mixture for 6 h and pour the resulting mixture into 1 l ice water. Collect and dry the resulting crystals.

### EXAMPLE 3

### 1,3-Dihydro-1-(4-pyrazolylmethyl)-2H-imidazole-2-thione

Under reflux conditions, heat a mixture of 9 g (0.14 mol) 4-pyrazolylcarboxaldehyde, 0.1 g TsOH, 18.6 g (0.14 mol) aminoacetaldehyde diethylacetal and 150 ml ethanol for 1-1/2 hours. Cool and concentrate reaction mixture, re-dissolve residue in 200 ml ethanol. Slowly add 5.7 g (0.15 mol) solid NaBH₄ and reflux the resulting mixture for 3 h, pour the mixture into water. Extract the intermediate aminoacetal derivative into CH₂Cl₂ (2x 150 ml). Dry (over Na₂SO₄) and concentrate the resulting mixture to give 32.1 g of a tan residue. Dissolve the entire residue in 150 ml ethanol and further add 15.5 g (0.16 mol) KSCN, 40 ml H₂O and 15 ml concentrated HCl. Reflux the mixture for 5 hrs., allow the mixture to cool and pour into 1 l of ice water and neutralize with dilute NaOH. Collect and dry the resulting crystals.

### EXAMPLE 4

### 1,3-Dihydro-1-(4-pyrimidinylmethyl)-2H-imidazole-2-thione

A mixture of 10.8 g (0.1 mol) 4-pyrimidinecarboxaldehyde, 13.1 g (0.1 mol) amino acetaldehyde diethylacetal, 0.1 g TsOH and 300 ml ethanol is placed in a 500 ml round bottomed flask. The reaction is refluxed for 2 hrs. then concentrated to give the crude imine. The imine is dissolved in 250 ml ethanol and 3.8 g (0.1 mol) solid NaBH₄ added slowly. After addition is completed, the mixture is refluxed 2 h. The reaction is diluted with H₂O to a total volume of 1 l and the amine derivative extracted into CH₂Cl₂ (2 X 250 ml). After drying (Na₂SO₄) and concentration, the amine is obtained as an orange oil. Without further purification, the amine is dissolved in 200 ml ethanol and 11.6 g (0.12 mol) KSCN, 40 ml water and 15 ml concentrated hydrochloric acid added. The reaction is gently refluxed for 6 h, cooled and poured onto ice. The desired product, as a crystalline solid is collected and dried in a vacuum oven.

### EXAMPLE 5

### 1,3-Dihydro-1-(2-(5-methylimidazolyl)methyl)-2H-imidazole-2-thione

A mixture of 11.0 g (0.1 mol) 4(5)-methyl-2-imidazolecarboxaldehyde, 13.3 g (0.1 mol) aminoacetaldehyde diethylacetal, 0.2 g TsOH and 200 ml ethanol is heated to reflux. After 1 h, the reaction is cooled and concentrated. The residue is dissolved in 200 ml ethanol and 3.8 g (0.1 mol), solid NaBH₄ added in small portions. The reaction is then refluxed for 2 h, poured onto 1.2 l H₂O and the product extracted into CH₂Cl₂ (2 X 200 ml). The CH₂Cl₂ solution is dried (Na₂SO₄) and concentrated to give a tan oil. The oil is dissolved in 200 ml ethanol and 11.6 g (0.12 mol) KSCN, 40 ml H₂O and 15 ml concentrated hydrochloric acid added. The reaction is refluxed for 6 h. After cooling, the reaction is poured onto 1 l ice. The product, tan crystals, is collected by vacuum filtration and dried.

### EXAMPLE 6

### 1,3-Dihydro-1-(2-furylmethyl)-2H-imidazole-2-thione

A 500 ml flask is charged with 38.4 g (0.4 mol) furan-2-carboxaldehyde, 53.2 g (0.4 mol) aminoacetaldehyde diethylacetal and 100 ml ethanol. The solution is refluxed for 1-1/2 h then concentrated under vacuum to give an orange oil. This oil is dissolved in 150 ml ethanol and 17 g (0.45 mol) NaBH₄ is added slowly. This mixture is refluxed 1-1/2 h, cooled and diluted with water. A little glacial acetic acid is added to destroy the excess NaBH₄. The product is extracted into EtOAc (2 X 300 ml). After drying (Na₂SO₄) and concentration, 70.3 g amino derivative is obtained; 21.3 g (0.1 mol) amino derivative is placed in a 500 ml flask and 11.7 g (0.12 mol) KSCN, 150 ml ethanol, 40 ml water and 15 ml concentrated hydrochloric acid is added. The reaction is refluxed for 5 h. The cooled reaction is diluted with water and the product extracted into EtOAc. Drying (Na₂SO₄) and concentration gives 14.7 g crude product. Purification by flash chromotography (4% MeOH/CH₂Cl₂) yields 4.75 g (26%) desired product, mp 105.5-108°C (EtOAc).

### EXAMPLE 7

### 1,3-Dihydro-1-(thienylmethyl)-2H-imidazole-2-thione

Under a blanket of nitrogen, 11.3 g (0.1 mol) 2-aminomethylthiophene is added to 19.6 g (0.11 mol) 1,1'-thiocarbonyldiimidazole in 200 ml anhydrous toluene at 0°C. The reaction is held at 0°C for 4 h. Then 10.5 g (0.1 mol) aminoacetaldehyde dimethyl acetal is added and the reaction is warmed at 80°C for 2 h. The toluene is removed and the residue dissolved in 100 ml ethanol, 15 ml water and 15 ml concentrated HCl. The mixture is refluxed 5 hrs., cooled and poured onto 1 l ice. After recrystallization (1/1 EtOH/H₂O) the desired product is obtained as white shiny crystals, mp 128-130°C.

### EXAMPLE 8

The compound of this example is not claimed here, but it is an intermediate in the procedure of Example 12 and is therefore useful to exemplify a preparation method according to the present disclosure.

### 1,3-Dihydro-1-(4-pyridyl)-2H-imidazole-2-thione

Under a blanket of nitrogen, 14.1 g (0.15 mol) 4-aminopyridine is added to 28.5 g (0.16 mol) 1,1'-thiocarbonyldiimidazole in 300 ml anhydrous DMF at 0°C. The reaction is held at 0°C for 4 h. To the 4-isothiocyanatopyridine formed 15.8 g (0.15 mol) aminoacetaldehyde dimethyl acetal is added and the reaction is warmed at 80°C for 24 h. The reaction is poured into water and extracted with ethyl acetate providing a dark residue on evaporation. This material is dissolved in 200 ml ethanol, 20 ml water and 20 ml concentrated hydrochloric acid. The mixture is refluxed for 5 h, cooled and poured onto 1 l ice. After purification, crystalline product is obtained as a tan solid.

### EXAMPLE 9

### 1,3-Dihydro-1-(2-thienyl)-2H-imidazole-2-thione

A mixture of 2-isothiocyanatothiophene (16.9 g, 0.12 mol), aminoacetaldehyde dimethyl acetal (12.6 g, 0.12 mol) in 200 ml toluene is refluxed for 2 h. After removal of the toluene, the residue is dissolved in 200 ml ethanol and 45 ml conc. HCl added. After the reaction is refluxed for 5 h, it is poured onto ice. The product is collected and purified by recrystallization.

In a similar manner, by following the generic teachings related to Reaction Schemes A to C and by substantially following the procedures of the foregoing examples, there may be prepared the following 1,3-dihydro-2H-imidazole-2-thiones:
5-chloro-1-(2-thienylmethyl)-1,3-dihydro-2H-imidazole-2-thiones,
5-bromo-1-(2-thienylmethyl)-1,3-dihydro-2H-imidazole-2-thiones,
5-methyl-1-(2-thienylmethyl)-1,3-dihydro-2H-imidazole-2-thiones,
4-phenyl-1-(2-thienylmethyl)-1,3-dihydro-2H-imidazole-2-thiones,
5-benzyl-1-(2-thienylmethyl)-1,3-dihydro-2H-imidazole-2-thiones,
5-cyano-1-(2-thienylmethyl)-1,3-dihydro-2H-imidazole-2-thiones,
5-fluoro-1-(2-thienylmethyl)-1,3-dihydro-2H-imidazole-2-thiones,
and their 1-(2-thienylethyl), 1-(2-thienylpropyl)-, 1-(2-thienylbutyl) and 1-(2-thienyl) homologs.

Similarly, the corresponding analogs of the foregoing may be prepared for the corresponding 1-position pyrazolyl, furyl, pyrimidinyl, pyrrolyl and imidazolyl substituted 1,3-dihydro-2H-imidazole-2-thiones.

### EXAMPLE 10

The compound of this example is not claimed here, but it is an intermediate in the procedure of Example 11 that in turn is relevant to the procedures of Examples 15, 16 and 18 and is therefore useful to exemplify a preparation method according to the present disclosure.

### 1-(3-Thienylmethyl)imidazole

Procedure: In a 3 neck, round-bottomed flask reflux a mixture of 11.6 g (0.17 mol) imidazole, 30 g (0.17 mol) 3-bromomethylthiophene, 46 g (0.33 mol) K₂CO₃ and 400 ml dry acetone. After refluxing for 4 hrs. filter the reaction mixture and wash the inorganic solids with acetone. Remove the acetone under vacuum and partition the residue between H₂O/EtOAc. Wash the EtOAc layer several times with H₂O, dry (Na₂SO₄) and concentrate the washed material to obtain a pale yellow oil.

### EXAMPLE 11

The compound of this example is not claimed here, but it is an intermediate in the procedure of Examples 15, 16 and 18 and is therefore useful to exemplify a preparation method according to the present disclosure.

### 2-Cyano-1-(3-thienylmethyl)imidazole

In a 250 ml, 4-neck flask equipped with a nitrogen bubbler, gas inlet tube, thermometer and septum, add 100 ml acetonitrile and bubble cyanogen chloride (15 g, 0.24 mol) into the acetonitrile. Cool the solution in an ice bath and add 8.2 g (0.05 mol) 1-(3-thienylmethyl)-2H-imidazole. After the colorless solution turns yellow-orange and about 1 h after an orange precipitate forms, cool the slurry orange precipitates to -20°C, slowly add 42 ml (0.3 mol) of triethylamine, holding the temperature below 0°C. After 1 h at room temperature, pour the reaction onto 600 ml saturated NaHCO₃ and extract with ether (3 X 150 ml). Dry and combine organic layers to obtain tan oil. The oil is purified via Kugelrohr distillation to obtain a colorless oil.

In a similar manner, by treating the 1-substituted-4X (or 5X) imidazoles of Example 12 according to the procedures of this example, there may be prepared the corresponding 2-cyano derivatives.

### EXAMPLE 12

The compound of this example is not claimed here. However, the following procedure is used to prepare some of the claimed compounds by converting the 2-thione derivatives obtained according to Examples 1-7 and 9 into the corresponding reduced compounds, thus obtaining the corresponding 1-substituted 4X (or 5X) imidazoles, wherein X represent the substituent in position 4 or 5 of these compounds.

### 1-(4-Pyridyl)imidazole

An ethanolic solution of 5.9 g (0.028 mol) 1,3-dihydro-1-(4-pyridyl)-2H-imidazole-2-thione prepared in Example 8 is charged with 23.0 g Raney nickel and 10 ml concentrated ammonium hydroxide. The black slurry is refluxed for 2 h, then filtered to remove the nickel catalyst. The ethanol is removed under vacuum and the product is extracted into CH₂Cl₂ (2 X 100 ml). Drying (Na₂SO₄) and concentration gives the 1-(4-pyridyl)imidazole.

### EXAMPLE 13

### Methyl-2-[1-(2-thienylmethyl)-imidazolyl]oxalate

A solution of 1-(2-thienylmethyl)imidazole (4 g, 0.024 mol) in 100 ml CH₃CN was charged with 3.0 g methyl oxalyl chloride. The solution was stirred for 1 h and then 4.1 ml (0.03 mols) Et₃N was added. The color changed and a white precipitate was formed. (TLC (60% EtOAc/hex) showed mainly one spot.) The mixture was diluted with H₂O and the product extracted into EtOAc (2 X 150 ml). After drying (Na₂SO₄) and concentration , 6.7 g of a brown oil was obtained. Flash chromatography (60% ethylacetate/hexane) gives 6.2 g of the desired product as an orange-yellow oil.

In a similar manner, by treating the 1-substituted-4X (or 5X) imidazole prepared according to the procedures of Example 12, the analogous alpha keto esters may be prepared, which compounds may also be converted to the analogous alpha keto acids by standard procedures.

### EXAMPLE 14

### 1-(2-Thienylmethyl)-2-(dimethylaminoethylthio)-imidazole

Stir a mixture of 8 g (0.041 mol) 1,3-dihydro-1-(2-thienylmethyl)-2H-imidazole -2-thione, 5.9 g (0.041 mol) 2-dimethylaminoethyl chloride hydrochloride, 200 ml ethanol and 17 ml 5N NaOH at ambient temperatures for 18 h. Remove the ethanol under vacuum and extract the product into Et₂O. Prepare the hydrochloride salt by adding etheral HCl. Collect and dry hygroscopic white solid.

In a similar manner, by substituting the reactant with the appropriate 2-thiones and by substantially following the procedure of this example there may be produced:
1-(2-thienylpropyl)-2-(dimethylaminoethylthio)-imidazole,
1-(3-thienylmethyl)-2-(dimethylaminoethylthio)-imidazole,
1-(4-pyrazolylmehtyl)-2-(dimethylaminoethylthio)-imidazole,
1-(1-methylpyrrol-2-ylmethyl)-2-(dimethylaminoethylthio)-imidazole,
1-(4-pyrimidinylmethyl)-2-(dimethylaminoethylthio)-imidazole,
1-(5-methylimidazol-2-ylmethyl)-2-(dimethylaminoethylthio)-imidazole,
1-(2-furanylmethyl)-2-(dimethylaminoethylthio)-imidazole,
1-(2-thienyl)-2-(dimethylaminoethylthio)-imidazole,
1-(3-thienyl)-2-(dimethylaminoethylthio)-imidazole,
1-(4-pyrazolyl)-2-(dimethylaminoethylthio)-imidazole,
1-(1-methyl-2-pyrrolyl)-2-(dimethylaminoethylthio)-imidazole,
1-(4-pyrimidinyl)-2-(dimethylaminoethylthio)-imidazole,
1-(2-furanyl)-2-(dimethylaminoethylthio)-imidazole,
as well as the des-alkylated aminoethylthio analogs of the foregoing.

### EXAMPLE 15

### 2-Aminomethyl-1-(3-furylmethyl)imidazole

Place 4.0 g (0.021 mol) 2-cyano-1(3-furanylmethyl)-2H-imidazole, 20 ml ethanolic HCl, 20 ml ethanol and 1 g 10% Pd/C in a Parr hydrogenator with an initial pressure of 3.95 bar (52 psi). After 18 h remove the reaction mixture from the Parr hydrogenator, add 20 ml H₂O, remove the catalyst by filtration, concentrate the filtrate to obtain an off-white solid which is recrystallized from ethanol containing a little concentrated HCl to give the desired product as a white solid.

In a similar manner, the 2-cyano derivatives prepared according to procedures of Example 11 may be converted to their 2-aminomethyl derivatives by following the procedure of this example.

### EXAMPLE 16

The compound of this example is not claimed here, but it is an intermediate in the procedure of Examples 19 and 20 and is therefore useful to exemplify a preparation method according to the present disclosure.

### Ethyl-2-([1-(3-furylmethyl)imidazole]methaneimidate)

Under a blanket of nitrogen, mechanically stir a solution of 2-cyano-1-(3-thienylmethyl)imidazole (10 g, 0.052 mol), absolute ethanol (2.4 g, 0.052 mol) and 500 ml chloroform is cooled to -10°C. Anhydrous HCl is sparged into the reaction for 1 h as the temperature is held below 0°C. Stir the reaction for 1 h at 0°C under dry nitrogen. The excess hydrogen chloride is removed by bubbling dry nitrogen through the reaction. Shake the chloroform solution with cold saturated sodium bicarbonate. After drying (Na₂SO₄) and concentration, the imidate is obtained as a colorless oil that rapidly crystallized.

In a similar manner, the 2-cyano derivatives prepared according to the procedures of Example 11 may be converted to their analogous imino esters by following the procedure of this example.

### EXAMPLE 17

### 1-(3-Thienylmethyl)-2-thioamidoimidazole

Hydrogen sulfide is sparged into a solution of 3.0 g (0.015 mol) of the amidine of Example 19 in 20 ml pyridine for 5 minutes. The reaction is warmed at 50°C for 8 h. After cooling, the majority of the pyridine is removed under vacuum. The residue is mixed with 200 ml cold water. The yellow solid is collected and dried.

### EXAMPLE 18

### 1-(3-Thienylmethyl)-2-amidoimidazole

A mixture of 5.0 g (0.026 mol) of the nitrile of Example 11 and 120 ml concentrated hydrochloric acid is placed in a 500 ml round bottomed flask. A small amount of ethanol is added to aid in solubility of the nitrile. The reaction is refluxed for 8 h and then the ethanol is distilled away. The white crystals are collected and dried.

In a similar manner, the 2-cyano derivatives preparable according to the procedures of Example 11 may be converted to their analogous 2-amido derivatives by following the procedures of this example.

### EXAMPLE 19

### 1-(3-Thienylmethyl)-2-imidazolemethanimidamide Hydrochloride

An ethanolic solution of 12 g (0.051 mol) imino ester of Example 16 is treated with 4.0 g (0.074 mol) NH₄Cl in 20 ml ethanol. The solution was stirred at room temperature for 7 h, then concentrated to give a white solid. Recrystallization (ethanol) gives the amidine hydrochloride salt as colorless crystals.

In a similar manner, the 2-imino esters prepared by the procedures of Example 16 (or their N-alkyl derivatives) may be converted to the analogous amidine derivatives by following the procedure of this example.

### EXAMPLE 20

### 1-(2-thienylmethyl)-2-imidazolemethanimidhydroxyamide Hydrochloride

A mixture of 2 g (0.0085 mol) of the imidate ester of Example 16 and 0.63 g (0.009 mol) hydroxylamine hydrochloride is stirred in 100 ml ethanol for 24 h. The reaction is diluted with water and the desired product as a white crystalline product, is collected and dried.

In a similar manner, the imidate esters preparable by the procedure of Example 17 may be converted to their corrresponding hydroxylamidines by following the procedure of this example.

The compounds of this invention exert valuable in vitro and in vivo pharmacological effects in that they are dopamine beta-hydroxylase (DBH) inhibitors and thus are expected to be valuable therapeutic agents useful in the treatment of hypertension.

The DBH inhibitory properties of the compounds of this invention can readily be determined in vitro by standard and well known procedures for assaying conversion of tyramine to octopamine in the presence of dopamine beta-hydroxylase. Enzymatic oxygenation by DBH is determined in aqueous solution in the presence of molecular oxygen, an electron donor such as ascorbate, and the necessary cofactors for the enzyme at pH of 5 and a temperature of 20°-40°C, preferably 37°C. The test compound is added at the desired concentration, and the system is incubated. Activity is measured by measuring the oxygen uptake using a polarographic electrode and an oxygen monitor by the method of S. May et al., J. Biol. Chem. 256, 2258 (1981). Inhibition is given in molar concentration of compound at which DBH activity was halved (IC ₅₀) when the test compounds were tested according to the above described procedure. The IC₅₀ (expressed in micromolar units) data of some of the compounds of this invention are expressed in Table I.

The compounds of this invention may also be tested for their in vivo DBH inhibiting property according to the procedure of Felice, Felice and Kessinger, J. Neurochem., 31, 1461-1465 (1978) wherein the effects on peripheral dopamine and norepinephrine levels are determined. In this test spontaneously hypertensive rats are dosed (i.p.) at 50 mg per kilogram of body weight and sacrificed three hours later. Average results, expressed in micrograms of dopamine (DA) per gram of heart tissue, are determined with the difference between the control and the treated rats being the in vivo (DBH) inhibitory effect of the test compound. Results on some of the compounds of this invention are shown in Table I.

The ability of the compounds of this invention to lower blood pressure can be determined in vivo using spontaneously hypertensive rats (SHR's) according to standard and well known procedures. The test compound is administered intraperitoneally (ip) to rats and the blood pressure monitored continuously. Since DBH is a major enzyme in the synthetic pathway of the catecholamines, it would be expected that the presence of an inhibitor would act to decrease the amount of catecholamines produced, and thereby have an antihypertensive effect. The results of the testing for this antihypertensive effect are shown in Table I.

**TABLE I**

| Inhibition of DBH In Vitro and In Vivo at 50 mg/kg, IP, 3 Hours Post Dose in SHR's* | | | |
|---|---|---|---|
| Compound | IC₅₀ (µM) | Heart DA (µg/g) | Max Change MBP (mmHg) |
| 1 | 5.4 | Control .024±.003 | -30±21^{a} (18%) |
| | | Treated .066±.005*** | |
| 2 | 1.6 | Control .024±.003 | -21±27 (12%) |
| | | Treated .066±.012*** | |
| 3 | 1.0 | Control .024±.003 | -- |
| | | Treated .079±.004*** | |
| 4 | 8.1 | Control .021±.002 | -22±5 (13%) |
| | | Treated .046±.009*** | |
| 5 | 6.0 | Control .021±.002 | -37±19 (10%) |
| | | Treated .048±.004*** | |
| 6 | 2.9 | Control .025±.007 | -42±17 (25%) |
| | | Treated .045±.005*** | |
| Compound 1 - 1,3-Dihydro-(2-thienylmethyl)-2H-imidazole-2-thione Compound 2 - 1,3-Dihydro-1-(3-thienylmethyl)-2H-imidazole-2-thione Compound 3 - 1-(2-Furanylmethyl)-1,3-dihydro-2H-imidazole-2-thione Compound 4 - 1-[(5-Chloro-2-thienyl)methyl]-1,3-dihydro-2H-imidazole-2-thione Compound 5 - 1,3-Dihydro-1-[(5-methyl-2-thienyl)methyl]-2H-imidazole-2-thione Compound 6 - 1-[(Fluoro-2-thienyl)methyl]-1,3-dihydro-2H-imidazole-2-thione | | | |

| | | | |
|---|---|---|---|
| * Spontaneously Hypertensive Rats. | | | |
| *** p<.001. | | | |
| a Mean Difference | | | |
| ± Standard Deviation. | | | |

Based on the foregoing test results, as well as by comparison with similar test results for compounds known to be useful, the compounds of this invention exert their DBH inhibiting effects (i.e., their IC₅₀ effects) at from 1 to 100 micromolar concentrations and are expected to exhibit end-use antihypertensive activity at doses of about 10 mg to 100 mg per kilogram of body weight.

As is true in most large classes of compounds suitable for use as chemotherapeutic applications, certain subgeneric groups and certain specific compounds will exhibit properties which render them more preferably than the entire class. In this instance it is expected that those compounds of formula I wherein X is hydrogen or lower alkyl, particularly methyl and ethyl are preferred, compounds wherein Y is SH or -SCH₂CH₂NH₂, -CH₂NH₂ or amidine are also preferred, compounds wherein n is 1 or 3 are preferred and compounds wherein the heterocycle is 2-or 3- thiophene or furan are preferred particularly when these heterocycles bear a halogen or lower alkyl function in the ring. Specifically preferred compounds are
2-[1-(2-thienylmethyl)-imidazolyl] oxalate;
2-aminomethyl-1-(3-thienylmethyl)imidazole;
1-(3-thienylmethyl)-2-imidazolemethanimidamide hydrochloride;
1,3-dihydro-1-(2-thienylmethyl)-2H-imidazole-2-thione;
1,3-dihydro-1-(2-furanylmethyl)-2H-imidazole-2-thione;
1-(2-thienylmethyl)-2-imidazolemethanimidhydroxyamide hydrochloride;
1,3-dihydro-1-(3-thienylmethyl)-2H-imidazole-2-thione; and
1,3-dihydro-1-(4-pyrazolylmethyl)-2H-imidazole-2-thione.

As stated above, the compounds of this invention are useful in the treatment of hypertension. In the management of hypertension, the compounds of this invention may be utilized in compositions such as tablets, capsules or elixirs for oral administration, suppositories for rectal administration, sterile solutions or suspensions for parenteral or intramuscular administration, and the like. The compounds of this invention can be administered to patients (animals and human) in need of such treatment in dosages that will provide optimal pharmceutical efficacy. Although the dose will vary from patient to patient depending upon the nature and severity of disease, the patient's weight, special diets then being followed by a patient, concurrent medication, and other factors which those skilled in the art will recognize, the dosage range will generally be about 10 to 100 mg per kilogram of patient body weight per day, which can be administered in single or multiple doses. Naturally these dose ranges can be adjusted on a unit basis as necessary to permit divided daily dosage and, as noted above, the dose will vary depending on the nature and severity of the disease, weight of patient, special diets and other factors.

Typically, these combinations can be formulated into pharmaceutical compositions according to standard procedures generally known in the art.

About 1 to 100 mg of a compound or mixture of compounds of Formula I or a physiologically acceptable salt is compounded with a physiologically acceptable vehicle carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acacia, corn starch or gelatin; an excipient such as microcrystalline cellulose; a disintegrating agent such as corn starch, pregelatinized starch, algenic acid and the like; a lubricant such as magnesium stearate; a sweetening agent such as sucrose, lactose or saccharin; a flavoring agent such as peppermint, oil of wintergreen or cherry. When the dosage unit form is a capsule, it may contain in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc., or a synthetic fatty vehicle like ethyl oleate or the like. Buffers, preservatives, anti-oxidants and the like can be incorporated as required.

## Claims (Claims for the following Contracting State(s): BE, CH, DE, FR, GB, IT, LI, LU, NL, SE, GR)

1. A compound of the formula 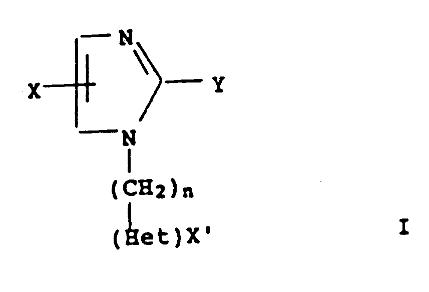 including the 2-thione tautomers thereof, and the non-toxic pharmaceutically acceptable salts thereof, wherein n is zero or the integer 1, 2, 3 or 4; X is hydrogen, cyano, C₁₋₆ lower alkyl, chloro, bromo, phenyl or benzyl, Z-substituted phenyl or benzyl with Z being C₁₋₆ lower alkyl or halogeno; Y is -CH₂NR₁R₂, -CONHR₁, -COCOOR₁, -CSNHR₁, 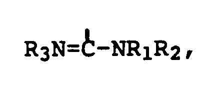 -SCH₂CH₂NR₁R₂ or - SR₁, wherein R₁ and R₂ independently represents hydrogen or C₁₋₆ alkyl and R₃ is hydrogen or hydroxy, with the proviso that when Y is -SR₁ or -SCH₂CH₂NR₁R₂, then X is hydrogen; (Het)X' is a heterocyclic moiety of the group thienyl, furyl, pyrimidinyl, pyrazolyl, pyrrolyl, and imidazol-2-yl, optionally substituted with either a halogen or a C₁₋₆ lower alkyl; for use as a medicine.

2. A compound of the formula 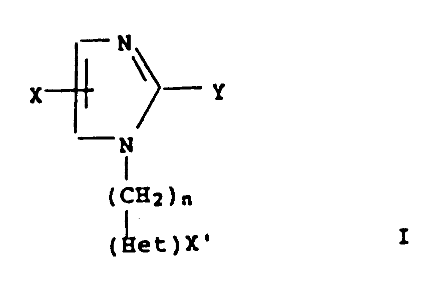 including the 2-thione tautomers therof, and the non-toxic pharmaceutically acceptable salts thereof, wherein n is zero or the integer 1, 2, 3 or 4; X is hydrogen, cyano, C₁₋₆ lower alkyl, chloro, bromo, phenyl or benzyl, Z-substituted phenyl or benzyl with Z being C₁₋₆ lower alkyl or halogeno; Y is -CH₂NR₁R₂, -CONHR₁, -COCOOR₁, -CSNHR₁, 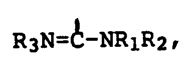 -SCH₂CH₂NR₁R₂ or - SR₁, wherein R₁ and R₂ independently represents hydrogen or C₁₋₆ alkyl and R₃ is hydrogen or hydroxy, with the proviso that when Y is -SR₁ or -SCH₂CH₂NR₁R₂, then X is hydrogen; (Het)X' is a heterocyclic moiety of the group consisting of thienyl, furyl, pyrimidinyl, pyrazolyl, pyrrolyl, and imidazol-2-yl, optionally substituted with either a halogen or a C₁₋₆ lower alkyl; with the proviso that when Y is SH and Het is 2-thienyl, then n is other than one.

3. Compounds of claim 1 or 2 wherein n is one.

4. Compounds of claim 1 or 2 wherein X is hydrogen.

5. Compounds of claim 1 or 2 wherein X is C₁₋₆ lower alkyl.

6. Compounds of claim 1 or 2 wherein Y is -SH or -SCH₂CH₂NH₂.

7. Compounds of claim 1 or 2 wherein Y is -CH₂NH₂.

8. Compounds of claim 1 or 2 wherein Het is 2- or 3-furyl.

9. Compounds of claim 8 wherein n is zero or one.

10. Compounds of claim 9 wherein Y is SH.

11. Compounds of claim 1 or 2 wherein Het is 2- or 3-thienyl.

12. Compounds of claim 11 wherein n is zero or one.

13. Compounds of claim 12 wherein Y is SH.

14. A compound of claim 1 or 2, said compound being 2-aminomethyl-1-(3-thienylmethyl)imidazole.

15. A compound of claim 1 or 2, said compound being 1-(3-thienylmethyl)-2-imidazolemethanimidamide hydrochloride.

16. A compound of claim 1 or 2, said compound being 1,3-dihydro-1-(2-furanylmethyl)-2H-imidazole-2-thione.

17. A compound of claim 1 or 2, said compound being 1-(2-thienylmethyl)-2-imidazolemethanimidhydroxyamide hydrochloride.

18. A compound of claim 1 wherein X and X' are hydrogen, Y is SH, n is 1 and Het is 2-thienyl.

19. Use of a compound of claim 1 for preparing a medicament for anti-hypertensive use.

20. Use of a compound of claim 1 wherein X and X' are hydrogen, Y is SH, n is 1 and Het is 2-thienyl for preparing a medicament for anti-hypertensive use.

21. A process for preparing a compound of claim 2 which comprises
a) the cyclization of a Schiff's base of the formula
X'-(Het)-(CH₂)ₙNHCHX²CH(O-C₁₋₆ alkyl)₂ (II)
wherein n is as defined in claim 1 but other than zero, X' is defined as in claim 1 and X² is as defined below, by the treatment with an aqueous acid in the presence of an alkali metal isothiocyanate to produce a compound of the formula 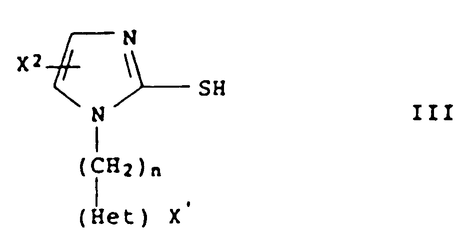 wherein n is other than zero; X² is hydrogen, cyano, C₁₋₆ alkyl, phenyl or benzyl, the phenyl or benzyl each optionally bearing a C₁₋₆ alkyl substituent,
b) the cyclization of a compound of the formula 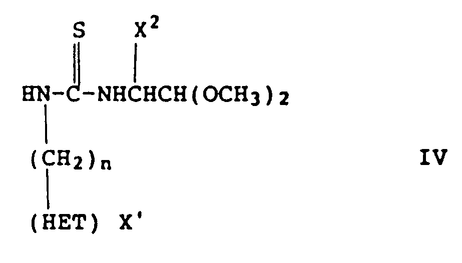 by treatment with an aqueous acid to produce a compound of the formula 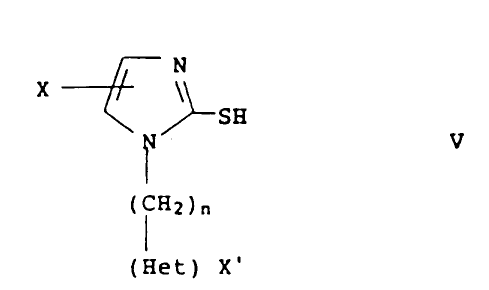
c) the alkylation of a compound of formula V by contacting with an aminoethylhalide of the formula R₁R₂NCH₂CH₂ halide at ambient temperature, in the presence of a base, to produce a compound of formula VI 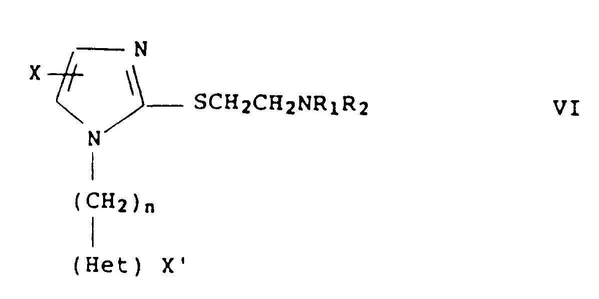
d) the cyanogenation of a compound of formula VII 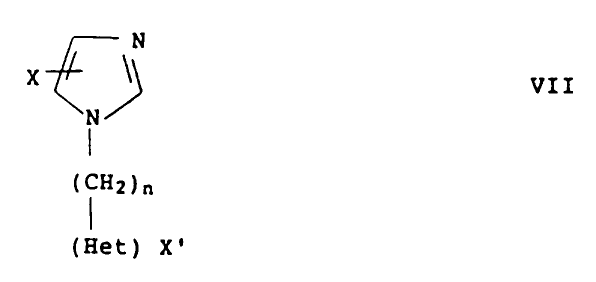 by treatment with a cyanogen halide, in an inert atmosphere, at room temperature followed by treatment with a base, in situ, of the so-obtained product, at temperatures below 0°C to prepare a compound of formula VIII 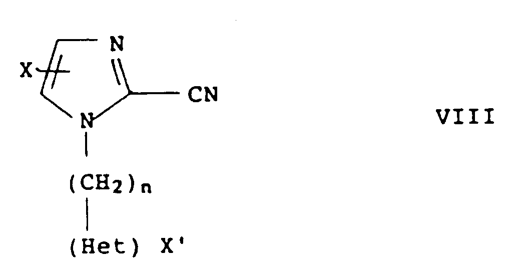
e) the conversion of the cyano moiety of a compound of formula VIII
(1) to its aminomethyl derivative by chemical reduction procedures, and optionally converting said amino methyl substituent to its corresponding N-alkyl derivatives by reaction with an appropriate aldehyde under Borsch reduction conditions,
(2) to its imidate ester derivative (i.e., 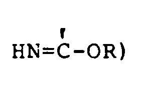 by reaction with an appropriate alcohol, followed by
(a) an optional conversion of the 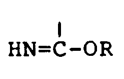 moiety to its corresponding amidino derivatives (i.e., 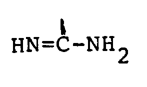 or 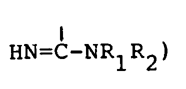 by reaction with the appropriate amine in an acidic medium, and optionally converting said amidino moieties to their corresponding thioamido derivatives (i.e., 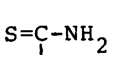 or S=CNR₁R₂) by reaction with H₂S in pyridine, or
(b) conversion of the 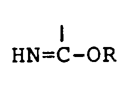 moiety to its hydroxy amidino (i.e., 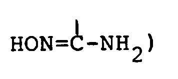 derivative which derivative optionally is converted to its alkylated derivative (i.e., 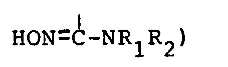 by treatment with an appropriate amine in the presence of 1 equivalent of acid,
(3) to its thioamide derivative (i.e., 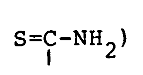 by reaction with H₂S in pyridine, and
(4) to its amide (i.e., CONH₂) by acid catalysis.

## Claims (Claims for the following Contracting State(s): AT, ES)

1. A process for preparing compounds of the general formula 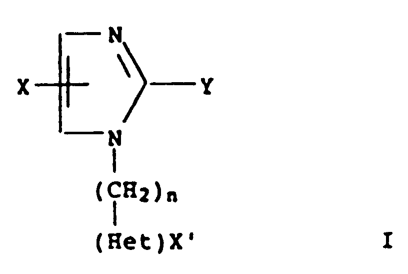 including the 2-thione tautomers, and the non-toxic pharmaceutically acceptable salts thereof, wherein n is zero or the integer 1, 2, 3 or 4, X is hydrogen, cyano, C₁₋₆ lower alkyl, chloro, bromo, phenyl, or benzyl, Z-substituted phenyl and benzyl with Z being C₁₋₆ lower alkyl or halogeno, Y is -CH₂NR₁R₂, -CONHR₁, -COCOOR₁, -CSNHR₁, 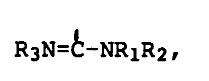 -SCH₂CH₂NR₁R₂ or - SR₁, wherein R₁ and R₂ independently represents hydrogen or C₁₋₆ alkyl and R₃ is hydrogen or hydroxy, with the proviso that when Y is -SR₁ or -SCH₂CH₂NR₁R₂, then X is hydrogen; (Het)X' is a heterocyclic moiety of the group consisting of thienyl, furyl, pyrazolyl, pyrimidinyl, pyrrolyl, and imidazol-2-yl, optionally substituted with either a halogen or a C₁₋₆ lower alkyl; with the proviso that when Y is SH and Het is 2-thienyl, n is other than 1, which comprises:
(a) the cyclization of a Schiff's base of the formula
X'-(Het)-(CH₂)ₙNHCHX²CH(O-C₁₋₆ alkyl)₂ (II)
wherein n is as above but other than zero, X' is as defined above and X² is as defined below,
by the treatment with an aqueous acid in the presence of an alkali metal isothiocyanate to produce a compound of the formula 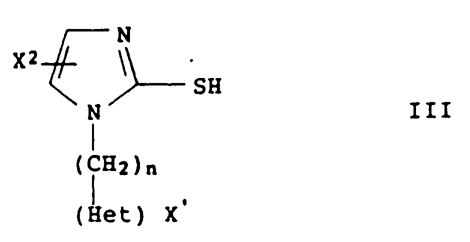 wherein n is other than zero; X² is hydrogen, cyano, C₁₋₆ alkyl, phenyl or benzyl, the phenyl or benzyl each optionally bearing a C₁₋₆ alkyl substituent,
b) the cyclization of a compound of the formula 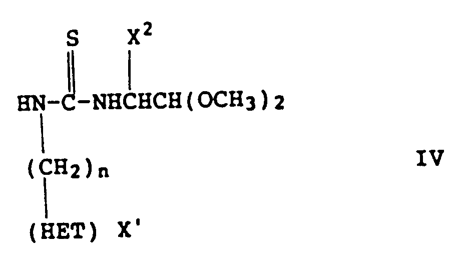 by treatment with an aqueous acid to produce a compound of the formula 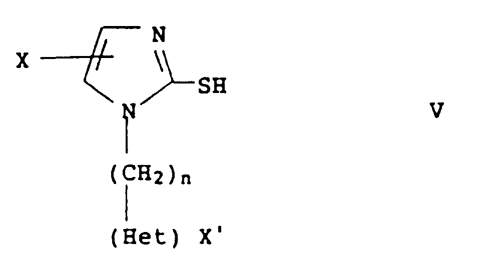
c) the alkylation of a compound of formula V by contacting with an aminoethylhalide of the formula R₁R₂NCH₂CH₂ halide at ambient temperature, in the presence of a base, to produce a compound of formula VI 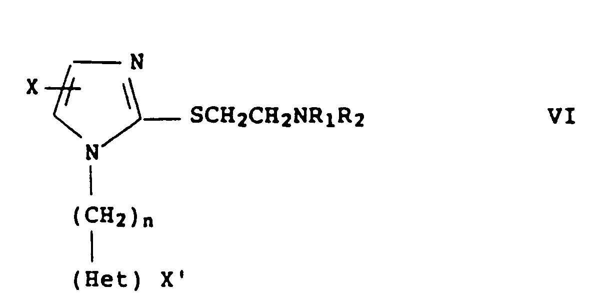
d) the cyanogenation of a compound of formula VII 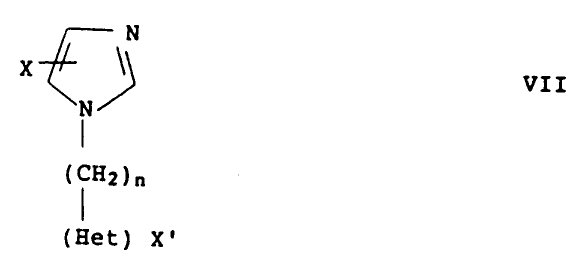 by treatment with a cyanogen halide, in an inert atmosphere, at room temperature followed by treatment with a base, in situ, of the so-obtained product, at temperatures below 0°C to prepare a compound of formula VIII 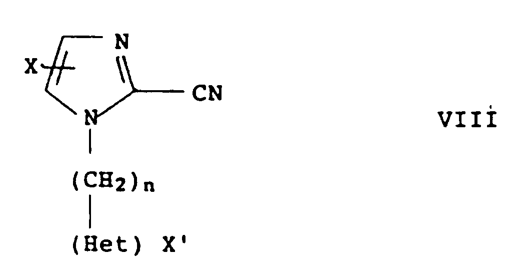
e) the conversion of the cyano moiety of a compound of formula VIII
(1) to its aminomethyl derivative by chemical reduction procedures, and optionally converting said amino methyl substituent to its corresponding N-alkyl derivatives by reaction with an appropriate aldehyde under Borsch reduction conditions,
(2) to its imidate ester derivative (i.e., 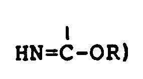 by reaction with an appropriate alcohol, followed by
(a) an optional conversion of the 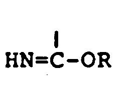 moiety to its corresponding amidino derivatives (i.e., 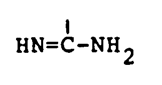 or 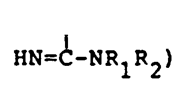 by reaction with the appropriate amine in an acidic medium, and optionally converting said amidino moieties to their corresponding thioamido derivatives (i.e., 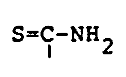 or S=CNR₁R₂) by reaction with H₂S in pyridine, or
(b) conversion of the 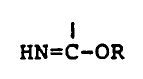 moiety to its hydroxy amidino (i.e., 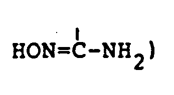 derivative which derivative optionally is converted to its alkylated derivative (i.e., 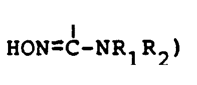 by treatment with an appropriate amine in the presence of 1 equivalent of acid,
(3) to its thioamide derivative (i.e., 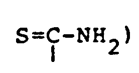 by reaction with H₂S in pyridine, and
(4) to its amide (i.e., CONH₂) by acid catalysis.

2. A process according to claim 1 for preparing a compound of formula I wherein n is one.

3. A process according to claim 1 or 2 for preparing a compound of formula I wherein X is hydrogen.

4. A process according to claim 1 or 2 for preparing a compound of formula I wherein X is C₁₋₆ lower alkyl.

5. A process according to claim 1 or 2 for preparing a compound of formula I wherein Y is -SH or -SCH₂CH₂NH₂.

6. A process according to claim 1 or 2 for preparing a compound of formula I wherein Y is -CH₂NH₂.

7. A process according to claim 1 or 2 for preparing a compound of formula I wherein Het is 2- or 3-furyl.

8. A process according to claim 1 for preparing a compound of formula I wherein n is zero or one and Het is 2- or 3-furyl.

9. A process according to claim 1 for preparing a compound of formula I wherein Y is SH, n is zero or one and Het is 2- or 3-furyl.

10. A process according to claim 1 for preparing a compound of formula I wherein Het is 2- or 3-thienyl.

11. A process according to claim 1 for preparing a compound of formula I wherein n is zero or one and Het is 2- or 3-thienyl.

12. A process according to claim 1 for preparing a compound of formula I wherein Y is SH, n is zero or one and Het is 2- or 3-thienyl.

13. A process according to claim 1 for preparing a compound of formula I, said compound being 2-aminomethyl-1-(3-thienylmethyl)imidazole.

14. A process according to claim 1 for preparing a compound of formula I, said compound being 1-(3-thienylmethyl)-2-imidazolemethanimidamide hydrochloride.

15. A process according to claim 1 for preparing a compound of formula I, said compound being 1,3-dihydro-1-(2-furanylmethyl)-2H-imidazole-2-thione.

16. A process according to claim 1 for preparing a compound of formula I, said compound being 1-(2-thienylmethyl)-2-imidazolemethanimidhydroxyamide hydrochloride.

17. Use of a compound of claim 1 for preparing a medicament for anti-hypertensive use.

18. Use of a compound of the general formula I according to claim 1 wherein X and X' are hydrogen, Y is SH, n is 1 and Het is 2-thienyl for preparing a medicament for anti-hypertensive use.

## Patentansprüche (Patentansprüche für folgende(n) Vertragsstaat(en): BE, CH, DE, FR, GB, IT, LI, LU, NL, SE, GR)

1. Verbindung der Formel 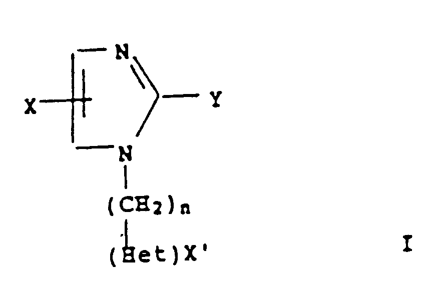 einschließlich deren 2-Thion-Tautomere und deren nicht toxischen pharmazeutisch verträglichen Salzen, wobei n gleich Null oder die ganze Zahl 1, 2, 3 oder 4 ist; X ein Wasserstoffatom, eine Cyangruppe, ein C₁₋₆ Niederalkylrest, ein Chloratom, ein Bromatom, eine Phenyl- oder Benzylgruppe, eine Z-substituierte Phenyl- oder Benzylgruppe ist, wobei Z ein C₁₋₆ Niederalkylrest oder Halogenatom ist; Y ein Rest -CH₂NR₁R₂, -CONHR₁, -COCOOR₁, -CSNHR₁, 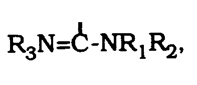 -SCH₂CH₂NR₁R₂ oder -SR₁ ist, wobei R₁ und R₂ unabhängig voneinander ein Wasserstoffatom oder einen C₁₋₆-Alkylrest darstellen und R₃ ein Wasserstoffatom oder eine Hydroxygruppe ist, mit der Maßgabe, daß X ein Wasserstoffatom ist, wenn Y ein Rest -SR₁ oder -SCH₂CH₂NR₁R₂ ist; (Het)X' ein heterocylischer Rest aus der Reihe der Thienyl-, Furyl-, Pyrimidinyl-, Pyrazolyl-, Pyrrolyl- und Imidazol-2-ylgruppen ist, gegebenenfalls mit entweder einem Halogenatom oder einem C₁₋₆ Niederalkylrest substituiert; zur Verwendung als Arzneimittel.

2. Verbindung der Formel 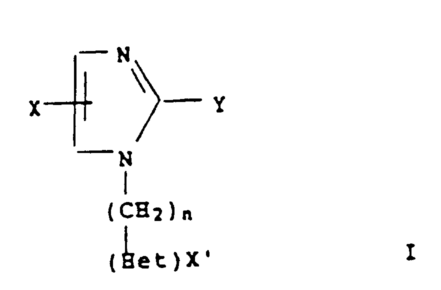 einschließlich deren 2-Thion-Tautomere und deren nicht toxischen pharmazeutisch verträglichen Salzen, wobei n gleich Null oder die ganze Zahl 1, 2, 3 oder 4 ist; X ein Wasserstoffatom, eine Cyangruppe, ein C₁₋₆ Niederalkylrest, ein Chloratom, ein Bromatom, eine Phenyl- oder Benzylgruppe, eine Z-substituierte Phenyl- oder Benzylgruppe ist, wobei Z ein C₁₋₆ Niederalkylrest oder Halogenatom ist; Y ein Rest -CH₂NR₁R₂, -CONHR₁, -COCOOR₁, -CSNHR₁, 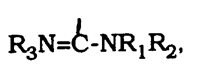 -SCH₂CH₂NR₁R₂ oder -SR₁ ist, wobei R₁ und R₂ unabhängig voneinander ein Wasserstoffatom oder einen C₁₋₆-Alkylrest darstellen und R₃ ein Wasserstoffatom oder eine Hydroxygruppe ist, mit der Maßgabe, daß X ein Wasserstoffatom ist, wenn Y ein Rest -SR₁ oder -SCH₂CH₂NR₁R₂ ist; (Het)X' ein heterocylischer Rest aus der Reihe der Thienyl-, Furyl-, Pyrimidinyl-, Pyrazolyl-, Pyrrolyl-und Imidazol-2-ylgruppen ist, gegebenenfalls mit entweder einem Halogenatom oder einem C₁₋₆ Niederalkylrest substituiert; mit der Maßgabe daß n ungleich Eins ist, wenn Y SH und Het eine 2-Thienylgruppe ist.

3. Verbindung nach Anspruch 1 oder 2, worin n gleich Eins ist.

4. Verbindung nach Anspruch 1 oder 2, worin X ein Wasserstoffatom ist.

5. Verbindung nach Anspruch 1 oder 2, worin X ein C₁₋₆ Niederalkylrest ist.

6. Verbindung nach Anspruch 1 oder 2, worin Y -SH oder -SCH₂CH₂NH₂ ist.

7. Verbindung nach Anspruch 1 oder 2, worin Y -CH₂NH₂ ist.

8. Verbindung nach Anspruch 1 oder 2, worin Het eine 2- oder 3-Furylgruppe ist.

9. Verbindung nach Anspruch 8, worin n gleich Null oder Eins ist.

10. Verbindung nach Anspruch 9, worin Y SH ist.

11. Verbindung nach Anspruch 1 oder 2, worin Het eine 2- oder 3-Thienylgruppe ist.

12. Verbindung nach Anspruch 11, worin n gleich Null oder Eins ist.

13. Verbindung nach Anspruch 12, worin Y SH ist.

14. Verbindung nach Anspruch 1 oder 2, wobei die Verbindung 2-Aminomethyl-1-(3-thienylmethyl)imidazol ist.

15. Verbindung nach Anspruch 1 oder 2, wobei die Verbindung 1-(3-Thienylmethyl)-2-imidazolmethanimidamid-Hydrochlorid ist.

16. Verbindung nach Anspruch 1 oder 2, wobei die Verbindung 1,3-Dihydro-1-(2-furanylmethyl)-2H-imidazol-2-thion ist.

17. Verbindung nach Anspruch 1 oder 2, wobei die Verbindung 1-(2-Thienylmethyl)-2-imidazolmethanimidhydroxamid-Hydrochlorid ist.

18. Verbindung nach Anspruch 1, wobei X und X' Wasserstoffatome sind, Y SH ist, n gleich 1 ist und Het eine 2-Thienylgruppe ist.

19. Verwendung einer Verbindung nach Anspruch 1 zur Herstellung eines blutdrucksenkenden Arzneimittels.

20. Verwendung einer Verbindung nach Anspruch 1, wobei X und X' Wasserstoffatome sind, Y SH ist, n gleich 1 ist und Het eine 2-Thienylgruppe ist, zur Herstellung eines blutdrucksenkenden Arzneimittels.

21. Verfahren zur Herstellung einer Verbindung nach Anspruch 2, umfassend:
a) die Cyclisierung einer Schiffbase der Formel
X'-(Het)-(CH₂)ₙNHCHX²CH(O-C₁₋₆Alkyl)₂ (II)
wobei n wie in Anspruch 1 definiert, aber ungleich Null ist, X' wie in Anspruch 1 definiert und X² wie nachstehend definiert ist, durch Behandlung mit einer wässerigen Säure in Gegenwart eines Alkalimetallisothiocyanats, wobei eine Verbindung der Formel 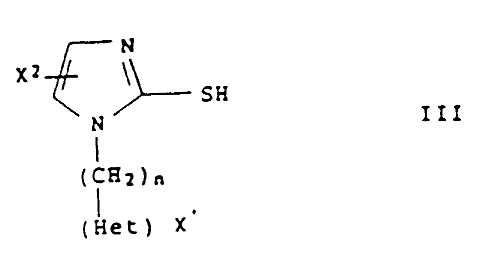 hergestellt wird, in der n ungleich Null ist; X² ein Wasserstoffatom, eine Cyanogruppe, ein C₁₋₆ Alkylrest, eine Phenyl- oder Benzylgruppe ist, wobei die Phenyl- oder Benzylgruppe jeweils gegebenenfalls einen C₁₋₆ Alkylrest trägt,
b) die Cyclisierung einer Verbindung der Formel 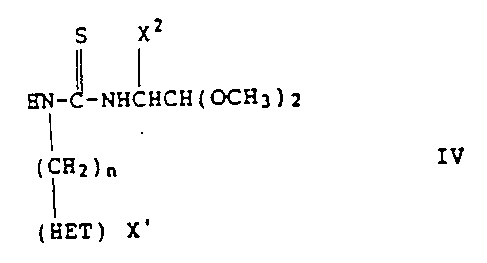 durch Behandlung mit einer wässerigen Säure, wobei man eine Verbindung der Formel 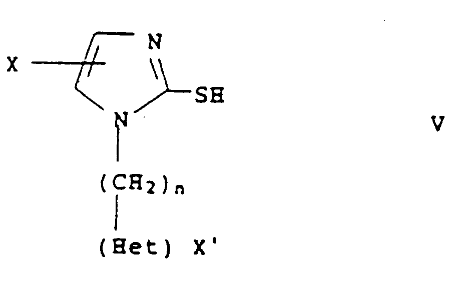 erhält,
c) die Alkylierung einer Verbindung der Formel V durch in Kontakt bringen mit einem Aminoethylhalogenid der Formel R₁R₂NCH₂CH₂-Halogen bei Raumtemperatur, in Gegenwart einer Base, wobei eine Verbindung der Formel VI 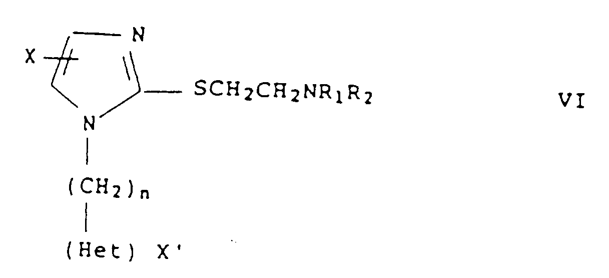 erhalten wird,
d) die Cyanierung einer Verbindung der Formel VII 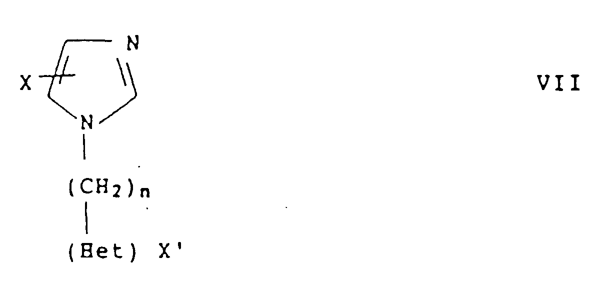 durch Behandlung mit Halogencyan in einer inerten Atmosphäre bei Raumtemperatur, gefolgt von in situ Behandlung mit einer Base des so erhaltenen Produkts, bei Temperaturen unter 0°C, wobei man eine Verbindung der Formel VIII erhält 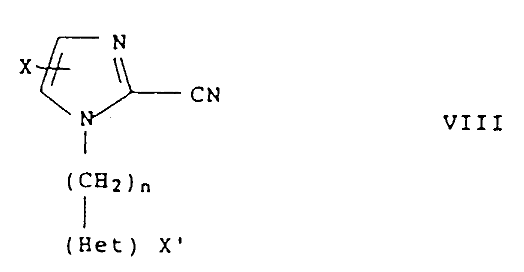
e) die Umwandlung des Cyanrests einer Verbindung der Formel VIII
(1) zu dessen Aminomethylderivat durch chemische Reduktionsverfahren und gegebenenfalls Umwandlung des Aminomethylsubstituenten in dessen entsprechende N-Alkylderivate durch Umsetzung mit einem geeigenten Aldehyd unter den Bedingungen der Borsch-Reduktion,
(2) zu dessen Imidatesterderivaten (das heißt 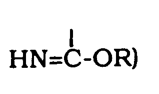 durch Umsetzung mit einem geeigneten Alkohol, gefolgt von
(a) gegebenenfalls einer Umwandlung des 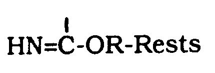 in dessen entsprechendes Amidinderivat (das heißt 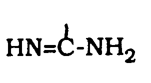 oder 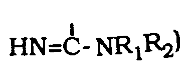 durch Umsetzung mit dem geeigneten Amin in einem sauren Medium und gegebenenfalls Umwandlung der Amidin-Reste in die entsprechenden Thioamidderivate (das heißt 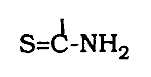 oder 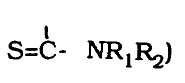 durch Umsetzung mit H₂S in Pyridin oder
(b) Umwandlung des 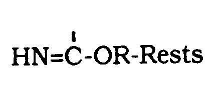 in dessen Hydroxyamidinderivat (das heißt 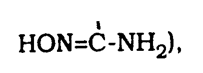 welches gegebenenfalls in dessen alkyliertes Derivat (das heißt 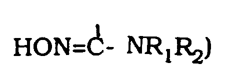 durch Behandlung mit einem geeigneten Amin in Gegenwart von 1 Äquivalent Säure umgewandelt wird,
(3) zu dessen Thioamidderivat (das heißt 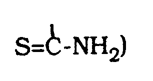 durch Umsetzung mit H₂S in Pyridin, und
(4) zu dessen Amid (das heißt CONH₂) durch saure Katalyse.

## Patentansprüche (Patentansprüche für folgende(n) Vertragsstaat(en): AT, ES)

1. Verfahren zur Herstellung einer Verbindung der allgemeinen Formel 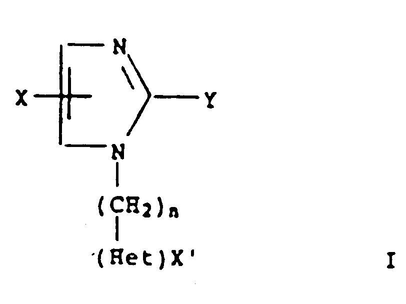 einschließlich deren 2-Thion-Tautomere und deren nicht toxischen pharmazeutisch verträglichen Salzen, wobei n gleich Null oder die ganze Zahl 1, 2, 3 oder 4 ist; X ein Wasserstoffatom, eine Cyangruppe, ein C₁₋₆ Niederalkylrest, ein Chloratom, ein Bromatom, eine Phenyl- oder Benzylgruppe, eine Z-substituierte Phenyl- oder Benzylgruppe ist, wobei Z ein C₁₋₆ Niederalkylrest oder Halogenatom ist; Y ein Rest -CH₂NR₁R₂, -CONHR₁, -COCOOR₁, -CSNHR₁, 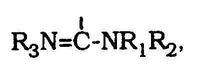 -SCH₂CH₂NR₁R₂ oder -SR₁ ist, wobei R₁ und R₂ unabhängig voneinander ein Wasserstoffatom oder einen C₁₋₆ Alkylrest darstellen und R₃ ein Wasserstoffatom oder eine Hydroxygruppe ist, mit der Maßgabe, daß X ein Wasserstoffatom ist, wenn Y ein Rest -SR₁ oder -SCH₂CH₂NR₁R₂ ist; (Het)X' ein heterocylischer Rest aus der Reihe der Thienyl-, Furyl-, Pyrazolyl-, Pyrimidinyl-, Pyrrolyl-und Imidazol-2-ylgruppen ist, gegebenenfalls entweder mit einem Halogenatom oder einem C₁₋₆ Alkylrest substituiert; mit der Maßgabe daß n ungleich Eins ist, wenn Y ein Rest SH und Het eine 2-Thienylgruppe ist; umfassend:
a) die Cyclisierung einer Schiffbase der Formel
X'-(Het)-(CH₂)ₙNHCHX²CH(O-C₁₋₆Alkyl)₂ (II)
wobei n wie vorstehend definiert, aber ungleich Null ist, X' wie vorstehend definiert und X² wie nachstehend definiert ist, durch Behandlung mit einer wässerigen Säure in Gegenwart eines Alkalimetallisothiocyanats, wobei eine Verbindung der Formel 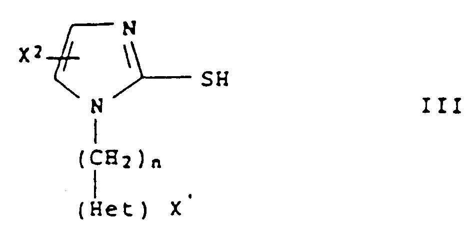 hergestellt wird, in der n ungleich Null ist; X² ein Wasserstoffatom, eine Cyanogruppe, ein C₁₋₆ Alkylrest, eine Phenyl- oder Benzylgruppe ist, wobei die Phenyl- oder Benzylgruppe jeweils gegebenenfalls einen C₁₋₆ Alkylrest trägt,
b) die Cyclisierung einer Verbindung der Formel 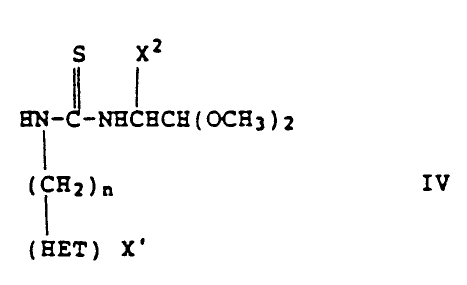 durch Behandlung mit einer wässerigen Säure, wobei man eine Verbindung der Formel 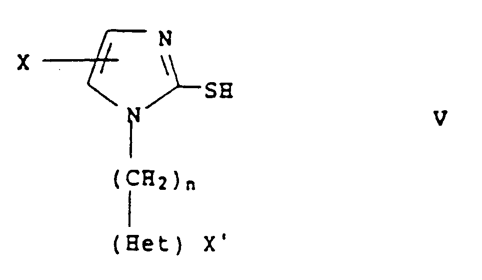 erhält,
c) die Alkylierung einer Verbindung der Formel V durch in Kontakt bringen mit einem Aminoethylhalogenid der Formel R₁R₂NCH₂CH₂-Halogen bei Raumtemperatur, in Gegenwart einer Base, wobei eine Verbindung der Formel VI 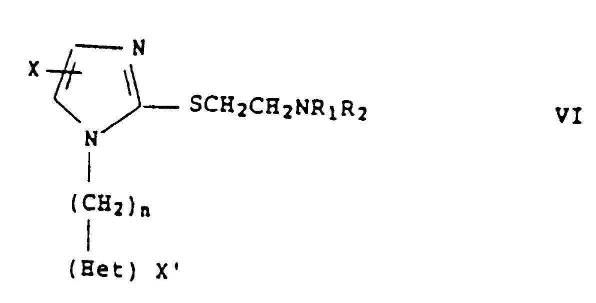 erhalten wird,
d) die Cyanierung einer Verbindung der Formel VII 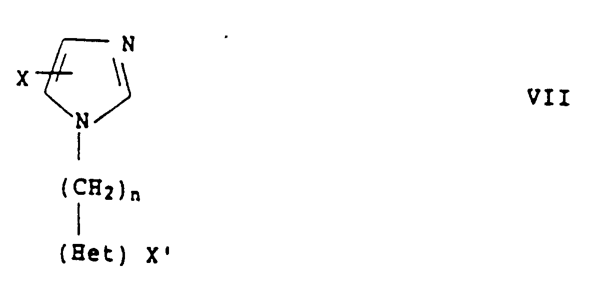 durch Behandlung mit Halogencyan in einer inerten Atmosphäre bei Raumtemperatur, gefolgt von in situ Behandlung mit einer Base des so erhaltenen Produkts, bei Temperaturen unter 0°C, wobei man eine Verbindung der Formel VIII erhält 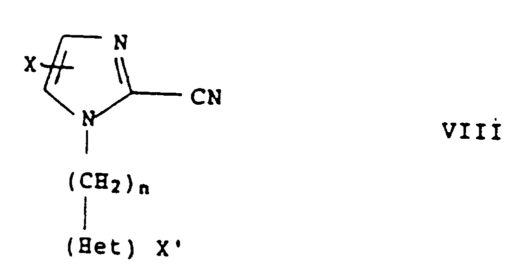
e) die Umwandlung des Cyanrests einer Verbindung der Formel VIII
(1) zu dessen Aminomethylderivat durch chemische Reduktionsverfahren und gegebenenfalls Umwandlung des Aminomethylsubstituenten in dessen entsprechende N-Alkylderivate durch Umsetzung mit einem geeigenten Aldehyd unter den Bedingungen der Borsch-Reduktion,
(2) zu dessen Imidatesterderivat (das heißt 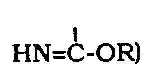 durch Umsetzung mit einem geeigneten Alkohol, gefolgt von
(a) gegebenenfalls einer Umwandlung des 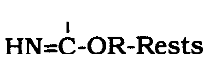 in dessen entsprechendes Amidinderivat (das heißt 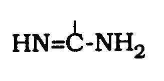 oder 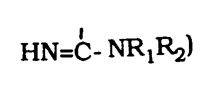 durch Umsetzung mit dem geeigneten Amin in einem sauren Medium und gegebenenfalls Umwandlung der Amidin-Reste in die entsprechenden Thioamidoderivate (das heißt 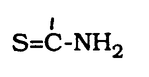 oder 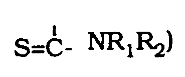 durch Umsetzung mit H₂S in Pyridin oder
(b) Umwandlung des 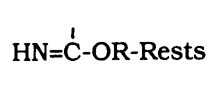 in dessen Hydroxyamidinderivat (das heißt 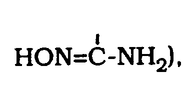 welches gegebenenfalls in dessen alkyliertes Derivat (das heißt 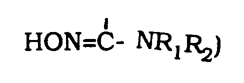 durch Behandlung mit einem geeigneten Amin in Gegenwart von 1 Äquivalent Säure umgewandelt wird,
(3) zu dessen Thioamidderivat (das heißt 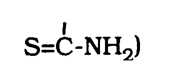 durch Umsetzung mit H₂S in Pyridin, und
(4) zu dessen Amid (das heißt CONH₂) durch saure Katalyse.

2. Verfahren nach Anspruch 1, zur Herstellung einer Verbindung der Formel I, in der n gleich Eins ist.

3. Verfahren nach Anspruch 1 oder 2, zur Herstellung einer Verbindung der Formel I, in der X ein Wasserstoffatom ist.

4. Verfahren nach Anspruch 1 oder 2, zur Herstellung einer Verbindung der Formel I, in der X ein C₁₋₆ Niederalkylrest ist.

5. Verfahren nach Anspruch 1 oder 2, zur Herstellung einer Verbindung der Formel I, in der Y -SH oder -SCH₂CH₂NH₂ ist.

6. Verfahren nach Anspruch 1 oder 2, zur Herstellung einer Verbindung der Formel I, in der Y -CH₂NH₂ ist.

7. Verfahren nach Anspruch 1 oder 2, zur Herstellung einer Verbindung der Formel I, in der Het eine 2- oder 3-Furylgruppe ist.

8. Verfahren nach Anspruch 1, zur Herstellung einer Verbindung der Formel I, in der n gleich Null oder Eins und Het eine 2- oder 3-Furylgruppe ist.

9. Verfahren nach Anspruch 1, zur Herstellung einer Verbindung der Formel I, in der Y SH, n gleich Null oder Eins und Het eine 2- oder 3-Furylgruppe ist.

10. Verfahren nach Anspruch 1, zur Herstellung einer Verbindung der Formel I, in der Het eine 2- oder 3-Thienylgruppe ist.

11. Verfahren nach Anspruch 1, zur Herstellung einer Verbindung der Formel I, in der n gleich Null oder Eins und Het eine 2- oder 3-Thienylgruppe ist.

12. Verfahren nach Anspruch 1, zur Herstellung einer Verbindung der Formel I, in der Y SH, n gleich Null oder Eins und Het eine 2- oder 3-Thienylgruppe ist.

13. Verfahren nach Anspruch 1, zur Herstellung einer Verbindung der Formel I, in der die Verbindung 2-Aminomethyl-1-(3-thienylmethyl)imidazol ist.

14. Verfahren nach Anspruch 1, zur Herstellung einer Verbindung der Formel I, in der die Verbindung 1-(3-Thienylmethyl)-2-imidazolmethanimidamid-Hydrochlorid ist.

15. Verfahren nach Anspruch 1, zur Herstellung einer Verbindung der Formel I, in der die Verbindung 1,3-Dihydro-1-(2-furanylmethyl)-2H-imidazol-2-thion ist.

16. Verfahren nach Anspruch 1, zur Herstellung einer Verbindung der Formel I, in der die Verbindung 1-(2-Thienylmethyl)-2-imidazolmethanimidhydroxamid-Hydrochlorid ist.

17. Verwendung einer Verbindung nach Anspruch 1 zur Herstellung eines blutdrucksenkenden Arzneimittels.

18. Verwendung einer Verbindung der allgemeine Formel I nach Anspruch 1, in der X und X' Wasserstoffatome sind, Y SH, n gleich 1 und Het eine 2-Thienylgruppe ist, zur Herstellung eines blutdrucksenkenden Arzneimittels.

## Revendications (Revendications pour l'(les) Etat(s) contractant(s) suivant(s): BE, CH, DE, FR, GB, IT, LI, LU, NL, SE, GR)

1. Un composé de formule 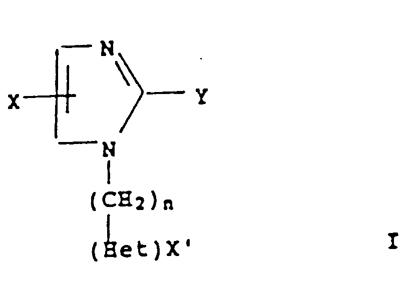 incluant les tautomères 2-thiones dudit composé, et ses sels pharmaceutiquement acceptables non toxiques, formule dans laquelle n est égal à 0 ou un nombre entier égal à 1, 2, 3 ou 4; X est un atome d'hydrogène, un groupe cyano, C₁₋₆-alkyle inférieur, chloro, bromo, phényle ou benzyle, phényle ou benzyle Z-substitué avec Z désignant un groupe C₁₋₆-alkyle inférieur ou halogéno ; Y est un groupe -CH₂NR₁R₂, -CONHR₁, -COCOOR₁, -CSNHR₁, 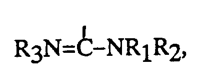 -SCH₂CH₂NR₁R₂ ou -SR₁, dans lequel R₁ et R₂ représentent indépendamment un atome d'hydrogène ou un groupe C₁₋₆-alkyle et R₃ est un atome d'hydrogène ou un groupe hydroxy, à la condition que, lorsque Y est -SR₁ ou -SCH₂CH₂NR₁R₂, alors X est un atome d'hydrogène ; (Het)X' est une portion hétérocyclique choisie dans le groupe suivant : thiényle, furyle, pyrimidinyle, pyrrazolyle, pyrrolyle et imidazole-2-yle, éventuellement substitué par un atome d'halogène ou par un groupe C₁₋₆-alkyle inférieur, à utiliser comme médicament.

2. Un composé de formule 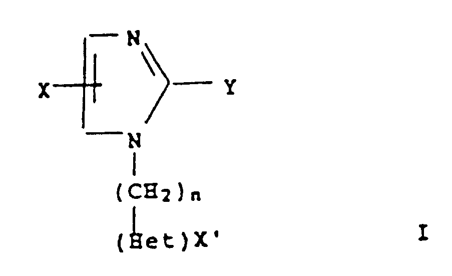 incluant ses tautomères 2-thiones, et ses sels pharmaceutiquement acceptables non toxiques, formule dans laquelle n est égal à 0 ou un nombre entier égal à 1, 2, 3 ou 4 ; X est un atome d'hydrogène, un groupe cyano, C₁₋₆-alkyle inférieur, chloro, bromo, phényle ou benzyle, phényle ou benzyle Z-substitué avec Z représentant un groupe C₁₋₆-alkyle inférieur ou un halogène ; Y est un groupe -CH₂NR₁R₂, -CONHR₁, -COCOOR₁, -CSNHR₁, 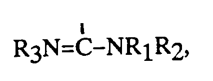 -SCH₂CH₂NR₁R₂ ou -SR₁, où R₁ et R₂ représentent indépendamment un atome d'hydrogène, ou un groupe C₁₋₆-alkyle et R₃ est un atome d'hydrogène ou un groupe hydroxy, à la condition que, lorsque Y est -SR₁ ou SCH₂CH₂NR₁R₂, alors X est un atome d'hydrogène ; (Het)X' est une portion hétérocyclique choisie dans le groupe constitué des groupes suivants : thiényle, furyle, pyrimidinyle, pyrazolyle, pyrrolyle et imidazole-2-yle, éventuellement substitué par un atome d'halogène ou par un groupe C₁₋₆-alkyle inférieur; à la condition que, lorsque Y est SH et Het est 2-thiényle, alors n est différent de un.

3. Composés selon la revendication 1 ou 2, selon laquelle n est égal à 1.

4. Composés selon la revendication 1 ou 2, selon laquelle X est un atome d'hydrogène.

5. Composés selon la revendication 1 ou 2, selon laquelle X est un groupe C₁₋₆-alkyle inférieur.

6. Composés selon la revendication 1 ou 2, selon laquelle X est -SH ou -SCH₂CH₂NH₂.

7. Composés selon la revendication 1 ou 2, selon laquelle Y est -CH₂NH₂.

8. Composés selon la revendication 1 ou 2, selon laquelle Het est 2- ou 3-furyle.

9. Composés selon la revendication 8, selon laquelle n est égal à 0 ou 1.

10. Composés selon la revendication 9, selon laquelle Y est SH.

11. Composés selon la revendication 1 ou 2, selon laquelle Het est 2- ou 3-thiényle.

12. Composés selon la revendication 11, selon laquelle n est égal à 0 ou 1.

13. Composés selon la revendication 12, selon laquelle Y est SH.

14. Un composé selon la revendication 1 ou 2, ledit composé étant le 2-aminométhyl-1-(3-thiénylméthyl)-imidazole.

15. Un composé selon la revendication 1 ou 2, ledit composé étant le chlorhydrate de 1-(3-thiénylméthyl)-2-imidazoleméthaneimidamide.

16. Un composé selon la revendication 1 ou 2, ledit composé étant la 1,3-dihydro-1-(2-furannylméthyl)-2H-imidazole-2-thione.

17. Un composé selon la revendication 1 ou 2, ledit composé étant le chlorhydrate de 1-(2-thiénylméthyl)-2-imidazoleméthaneimidehydroxyamide.

18. Un composé selon la revendication 1, selon lequel X et X' sont des atomes d'hydrogène, Y est SH, n est égal à 1 et Het est 2-thiényle.

19. Utilisation d'un composé selon la revendication 1 pour la préparation d'un médicament anti-hypertenseur.

20. Utilisation d'un composé selon la revendication 1, selon laquelle X et X' sont des atomes d'hydrogène, Y est SH, n est égal à 1 et Het est 2-thiényle pour la préparation d'un médicament anti-hypertenseur.

21. Un procédé de préparation d'un composé selon la revendication 2, qui comprend :
a) la cyclisation d'une base de Schiff de formule :
X'-(Het)-(CH₂)ₙNHCHX²CH(O-C₁₋₆-alkyle)₂ (II)
dans laquelle n est comme défini dans la revendication 1, mais est différent de zéro, X' est défini comme dans la revendication 1 et X² est comme défini ci-après, par le traitement par un acide aqueux en présence d'un isothiocyanate de métal alcalin pour produire un composé de formule : 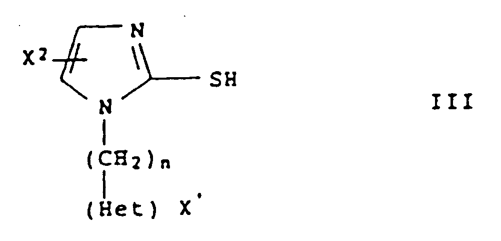 dans laquelle n est différent de zéro ; X² est un atome d'hydrogène, un groupe cyano, C₁₋₆-alkyle, phényle ou benzyle, le groupe phényle ou benzyle portant éventuellement un substituant C₁₋₆-alkyle,
b) la cyclisation d'un composé de formule : 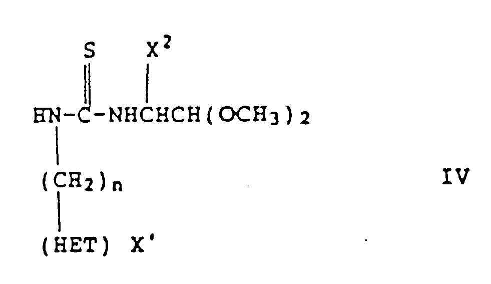 par traitement par un acide aqueux pour produire un composé de formule : 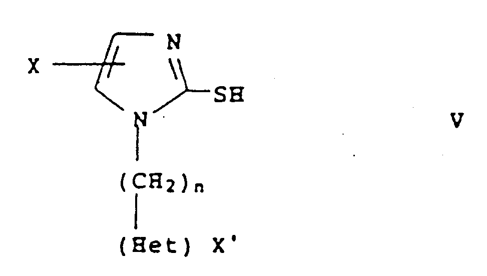
c) l'alkylation d'un composé de formule V par mise en contact avec un halogénure d'aminoéthyle de formule R₁R₂NCH₂CH₂-halogénure à la température ambiante, en présence d'une base, pour produire un composé de formule VI : 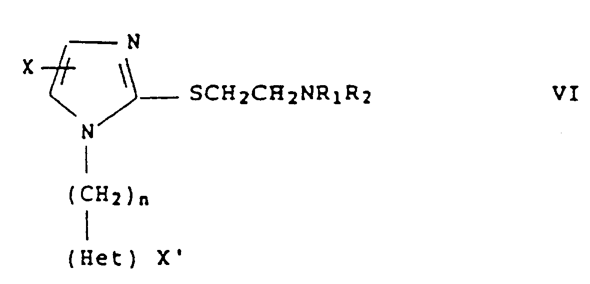
d) la cyanogénation d'un composé de formule VII 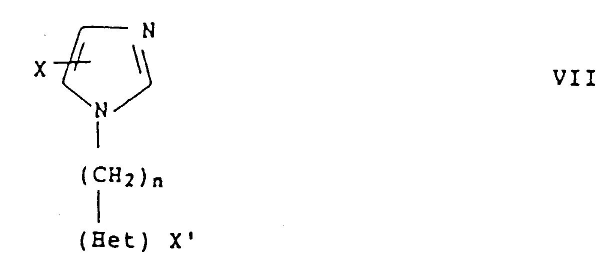 par traitement par un halogénure de cyanogène, dans une atmosphère inerte, à la température ambiante, suivi d'un traitement par une base in situ, du produit ainsi obtenu, à des températures inférieures à 0°C pour préparer un composé de formule VIII : 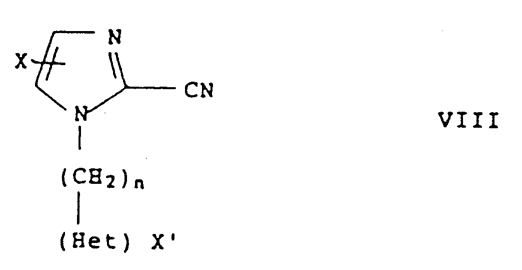
e) la conversion de la portion cyano d'un composé de formule VIII
(1) en son dérivé aminométhyle par des procédés de réduction chimique, et éventuellement conversion dudit substituant aminométhyle en dérivé correspondant N-alkyle par réaction avec un aldéhyde dans les conditions de réduction de Borsch,
(2) en son dérivé ester-imidate (c'est-à-dire 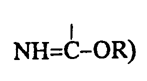 par réaction avec un alcool approprié suivie de
(a) une conversion éventuelle de la portion 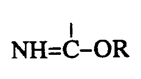 en dérivé amidino correspondant (c'est-à-dire 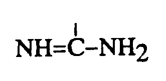 ou 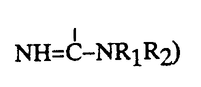 par réaction avec l'amine appropriée dans un milieu acide, et éventuellement conversion desdits portions amidino en leurs dérivés thioamido correspondants (c'est-à-dire 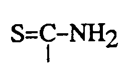 ou S=CNR₁R₂) par réaction avec H₂S dans la pyridine, ou
(b) conversion de la portion 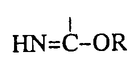 en son dérivé hydroxyamidino (c'est-à-dire 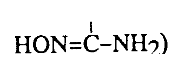 qui est éventuellement converti en son dérivé alkylé (c'est-à-dire 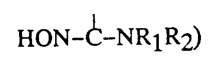 par traitement par une amine appropriée en présence d'un équivalent d'acide,
(3) en son dérivé thioamide (c'est-à-dire 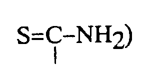 par réaction avec H₂S dans la pyridine, et
(4) en son amide (c'est-à-dire CONH₂) par catalyse acide.

## Revendications (Revendications pour l'(les) Etat(s) contractant(s) suivant(s): AT, ES)

1. Un procédé de préparation des composés de formule générale : 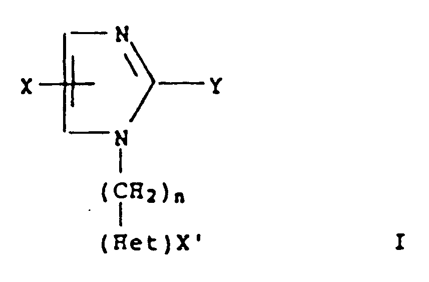 incluant les tautomères 2-thiones dudit composé, et ses sels pharmaceutiquement acceptables non toxiques, formule dans laquelle n est égal à 0 ou un nombre entier égal à 1, 2, 3 ou 4 ; X est un atome d'hydrogène, un groupe cyano, C₁₋₆-alkyle inférieur, chloro, bromo, phényle ou benzyle, phényle ou benzyle Z-substitué avec Z désignant un groupe C₁₋₆-alkyle inférieur ou halogéno ; Y est un groupe -CH₂NR₁R₂, -CONHR₁, -COCOOR₁, -CSNHR₁, R₃N=C-NR₁R₂, -SCH₂CH₂NR₁R₂ ou -SR₁, dans lequel R₁ et R₂ représentent indépendamment un atome d'hydrogène ou un groupe C₁₋₆-alkyle et R₃ est un atome d'hydrogène ou un groupe hydroxy, à la condition que, lorsque Y est -SR₁ ou -SCH₂CH₂NR₁R₂, alors X est un atome d'hydrogène ; (Het)X' est une portion hétérocyclique choisie dans le groupe suivant : thiényle, furyle, pyrimidinyle, pyrrazolyle, pyrrolyle et imidazole-2-yle, éventuellement substitué par un atome d'halogène ou par un groupe C₁₋₆-alkyle inférieur ; à la condition que, lorsque Y est SH et Het est 2-thiényle, alors n est différent de un, ledit procédé comprenant :
a) la cyclisation d'une base de Schiff de formule :
X'-(Het)-(CH₂)ₙNHCHX²CH(O-C₁₋₆-alkyle)₂ (II)
dans laquelle n est comme ci-dessus, mais est différent de zéro, X' est défini comme ci-dessus, et X² est comme défini ci-après, par le traitement par un acide aqueux en présence d'un isothiocyanate de métal alcalin pour produire un composé de formule : 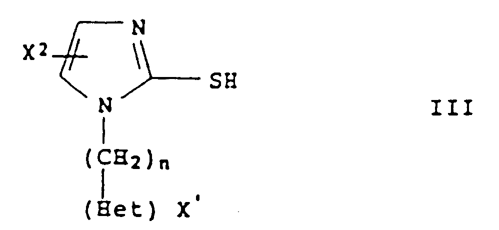 dans laquelle n est différent de zéro ; X² est un atome d'hydrogène, un groupe cyano, C₁₋₆-alkyle, phényle ou benzyle, le groupe phényle ou benzyle portant éventuellement un substituant C₁₋₆-alkyle,
b) la cyclisation d'un composé de formule : 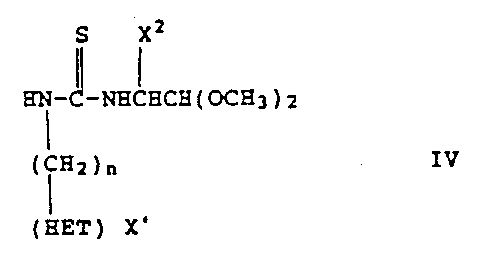 par traitement par un acide aqueux pour produire un composé de formule : 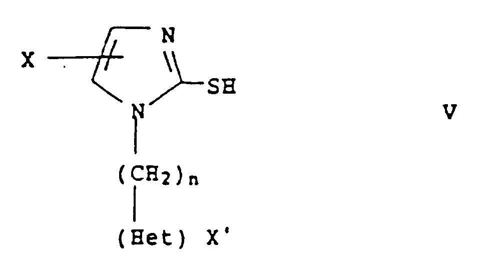
c) l'alkylation d'un composé de formule V par mise en contact avec un halogénure d'aminoéthyle de formule R₁R₂NCH₂CH₂-halogénure à la température ambiante, en présence d'une base, pour produire un composé de formule VI : 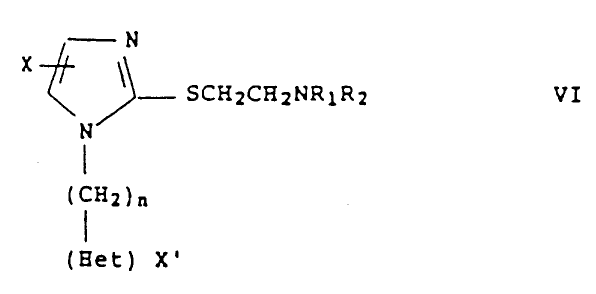
d) la cyanogénation d'un composé de formule VII 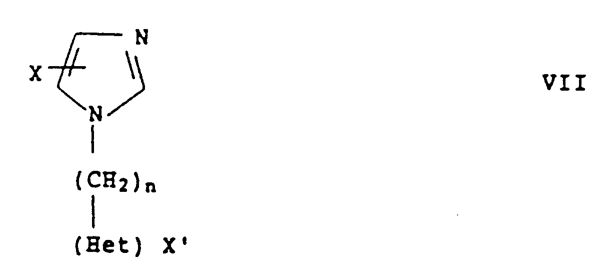 par traitement par un halogénure de cyanogène, dans une atmosphère inerte, à la température ambiante, suivi d'un traitement par une base in situ, du produit ainsi obtenu, à des températures inférieures à 0°C pour préparer un composé de formule VIII: 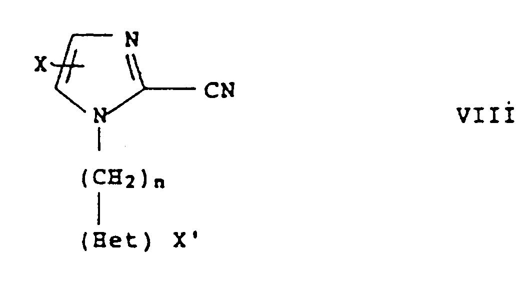
e) la conversion de la portion cyano d'un composé de formule VIII
(1) en son dérivé aminométhyle par des procédés de réduction chimique, et éventuellement conversion dudit substituant aminométhyle en dérivé correspondant N-alkyle par réaction avec un aldéhyde dans les conditions de réduction de Borsch,
(2) en son dérivé ester-imidate (c'est-à-dire 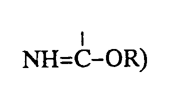 par réaction avec un alcool approprié suivie de
(a) une conversion éventuelle de la portion 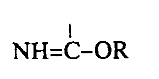 en dérivé amidino correspondant (c'est-à-dire 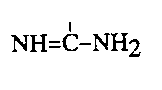 ou 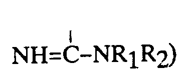 par réaction avec l'amine appropriée dans un milieu acide, et éventuellement conversion desdits portions amidino en leurs dérivés thioamido correspondants (c'est-à-dire S=C-NH₂ ou S=CNR₁R₂) par réaction avec H₂S dans la pyridine, ou
(b) conversion de la portion 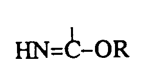 en son dérivé hydroxyamidino (c'est-à-dire 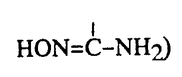 qui est éventuellement converti en son dérivé alkylé (c'est-à-dire 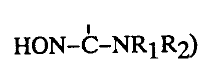 par traitement par une amine appropriée en présence d'un équivalent d'acide,
(3) en son dérivé thioamide (c'est-à-dire 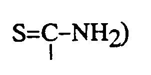 par réaction avec H₂S dans la pyridine, et
(4) en son amide (c'est-à-dire CONH₂) par catalyse acide.

2. Un procédé selon la revendication 1 pour la préparation d'un composé de formule I dans laquelle n est égal à 1.

3. Un procédé selon la revendication 1 ou 2 pour la préparation d'un composé de formule I dans laquelle X est un atome d'hydrogène.

4. Un procédé selon la revendication 1 ou 2 pour la préparation d'un composé de formule I dans laquelle X est un groupe C₁₋₆-alkyle inférieur.

5. Un procédé selon la revendication 1 ou 2 pour la préparation d'un composé de formule I dans laquelle Y est -SH ou -SCH₂CH₂NH₂.

6. Un procédé selon la revendication 1 ou 2 pour la préparation d'un composé de formule I dans laquelle Y est -CH₂NH₂.

7. Un procédé selon la revendication 1 ou 2 pour la préparation d'un composé de formule I dans laquelle Het est 2-ou 3-furyle.

8. Un procédé selon la revendication 1 pour la préparation d'un composé de formule I dans laquelle n est égal à 0 ou 1 et Het est 2- ou 3-furyle.

9. Un procédé selon la revendication 1 pour la préparation d'un composé de formule I dans laquelle Y est SH, n est égal à 0 ou 1 et Het est 2-ou 3-furyle.

10. Un procédé selon la revendication 1 pour la préparation d'un composé de formule I dans laquelle Het est 2- ou 3-thiényle.

11. Un procédé selon la revendication 1 pour la préparation d'un composé de formule I dans laquelle n est égal à 0 ou 1 et Het est 2-ou 3-thiényle.

12. Un procédé selon la revendication 1 pour la préparation d'un composé de formule I dans laquelle Y est SH, n est égal à 0 ou 1 et Het est 2-ou 3-thiényle.

13. Un procédé selon la revendication 1 pour la préparation d'un composé de formule I, ledit composé étant le 2-aminométhyl-1-(3-thiénylméthyl)imidazole.

14. Un procédé selon la revendication 1 pour la préparation d'un composé de formule I, ledit composé étant le chlorhydrate de 1-(3-thiénylméthyl)-2-imidazoleméthaneimidamide.

15. Un procédé selon la revendication 1 pour la préparation d'un composé de formule I, ledit composé étant la 1,3-dihydro-1-(2-furannylméthyl)-2H-imidazole-2-thione.

16. Un procédé selon la revendication 1 pour la préparation d'un composé de formule I, ledit composé étant le chlorhydrate de 1-(2-thiénylméthyl)-2-imidazoleméthaneimidehydroxyamide.

17. Utilisation d'un composé selon la revendication 1 pour la préparation d'un médicament anti-hypertenseur.

18. Utilisation d'un composé de formule générale I selon la revendication 1, selon laquelle X et X' sont des atomes d'hydrogène, Y est SH, n est égal à 1 et Het est 2-thiényle pour la préparation d'un médicament anti-hypertenseur.
